# FASCICULE DE BREVET EUROPEEN

(11) **EP 3 403 100 B1**
(45) Date de publication et mention de la délivrance du brevet: **11.03.2020**
(21) Numéro de dépôt: 17706533.1
(22) Date de dépôt: 16.01.2017
(51) Int. Cl.: G01N 33/576, G01N 33/68

(54) **PROCÉDÉ DE DIAGNOSTIC IN VITRO D'ATTEINTES HÉPATIQUES**
VERFAHREN ZUR IN-VITRO-DIAGNOSE VON LEBERERKRANKUNGEN
PROCESS FOR IN VITRO DIAGNOSIS OF HEPATIC DISORDERS

(30) Priorité: 15.01.2016 FR 1600081; 11.03.2016 FR 1652087
(43) Date de publication de la demande: 21.11.2018
(73) Titulaire: Commissariat à l'Énergie Atomique et aux Énergies Alternatives, 75015 Paris (FR); Centre Hospitalier Universitaire Grenoble Alpes, 38700 La Tronche (FR); Inserm, 75013 Paris (FR); Université Grenoble Alpes, 38400 Saint-Martin-d'Hères (FR); Université Paris-Sud, 91405 Orsay (FR)
(72) Inventeur: BRUN, Virginie, 38180 Seyssins (FR); GARIN, Jérôme, 38700 Corenc (FR); BRULEY, Christophe, 38450 Le Gua (FR); FAIVRE, Jamila, 75016 Paris (FR); ZARSKI, Jean-Pierre, 38330 ST Nazaire les Eymes (FR); LEROY, Vincent, 38640 Claix (FR)
(74) Mandataire: Plasseraud IP
(86) Numéro de dépôt international: PCT/FR2017/050089
(87) Numéro de publication internationale: WO 2017/121974

(56) Documents cités:
- WO-A2-2015/071669
- US-A1- 2015 147 761
- AN AFIDA ASHLA ET AL: "Genetic analysis of expression profile involved in retinoid metabolism in non-alcoholic fatty liver disease", HEPATOLOGY RESEARCH, vol. 40, no. 6, 25 mai 2010 (2010-05-25), pages 594-604, XP055365191, NL ISSN: 1386-6346, DOI: 10.1111/j.1872-034X.2010.00646.x
- RAGHOTHAMA CHAERKADY ET AL: "A Quantitative Proteomic Approach for Identification of Potential Biomarkers in Hepatocellular Carcinoma", JOURNAL OF PROTEOME RESEARCH, vol. 7, no. 10, 3 octobre 2008 (2008-10-03), pages 4289-4298, XP055007768, ISSN: 1535-3893, DOI: 10.1021/pr800197z
- SEUNGMOOK LEE ET AL: "Enhanced peptide quantification using spectral count clustering and cluster abundance", BMC BIOINFORMATICS, BIOMED CENTRAL, LONDON, GB, vol. 12, no. 1, 28 octobre 2011 (2011-10-28), page 423, XP021111478, ISSN: 1471-2105, DOI: 10.1186/1471-2105-12-423
- BRUN V ET AL: "Isotope dilution strategies for absolute quantitative proteomics", JOURNAL OF PROTEOMICS, ELSEVIER, AMSTERDAM, NL, vol. 72, no. 5, 21 juillet 2009 (2009-07-21), pages 740-749, XP026653191, ISSN: 1874-3919, DOI: 10.1016/J.JPROT.2009.03.007 [extrait le 2009-03-31] cité dans la demande

## Description

### Domaine de l'invention

L'invention appartient au domaine de l'hépatologie (médecine du foie) et concerne l'utilisation de biomarqueurs permettant de diagnostiquer des troubles hépatiques. Plus précisément, cette invention concerne des méthodes de diagnostic *in vitro* et/ou de suivi et/ou de pronostic *in vitro* des atteintes hépatiques telles que les insuffisances hépatiques aiguës et fulminantes ou la stéatohépatite par la détection et/ou le dosage de certains biomarqueurs.

### État de la technique

Les atteintes hépatiques regroupent une multitude de maladies affectant le foie de manière chronique (comme la cirrhose) ou aigüe (on parle alors d'insuffisance hépatique aigüe). Leurs causes sont variées et incluent l'alcoolisme, les virus des hépatites A, B, C et E, des anomalies génétiques (maladie de Wilson) ou immunitaires (hépatites auto-immunes), des intoxications médicamenteuses ou une surcharge graisseuse associée à l'obésité.

Les insuffisances hépatiques aiguës (« Acute Liver Injury», abrégé ici ALI) ou fulminantes (« Acute Liver Failure », abrégé ici ALF) se déclarent de manière soudaine chez des patients sans maladie hépatique préexistante. Leurs causes sont variées : médicaments (dont le paracétamol), virus, maladie auto-immune. Les insuffisances hépatiques aiguës se caractérisent par la mort des cellules hépatiques (hépatocytes) sur un mode nécrotique et/ou apoptotique et conduisent à une diminution de la masse fonctionnelle du foie. Ces atteintes hépatiques sont associées à des troubles de la coagulation sanguine et la modification des paramètres d'exploration clinico-biologique du foie (augmentation des transaminases et de la CK18 sérique). Dans leur forme la plus sévère (hépatite fulminante ou ALF), une encéphalopathie est également présente. Les atteintes hépatiques aiguës peuvent conduire à la mort du patient sans prise en charge médicale adaptée.

Les maladies du foie gras non induites par l'alcool (connues sous le sigle NAFLD qui est l'abréviation de « Non-Alcoholic Fatty Liver Disease ») sont des maladies hépatiques très fréquentes. Elles englobent un large éventail de maladies telles que la stéatose isolée (connue sous le sigle NAFL - « Non-Alcoholic Fat Liver »), la stéatohépatite non alcoolique (NASH - « Non-Alcoholic SteatoHepatitis ») et la cirrhose. Elles sont associées à une accumulation anormale de graisse dans le foie. Elles sont liées à l'augmentation de la prévalence du diabète et de l'obésité. La stéatohépatite non alcoolique (NASH) est une maladie potentiellement grave pouvant évoluer en cirrhose et entrainer une insuffisance hépatique chronique ou aigüe et/ou un carcinome hépatocellulaire.

Les atteintes hépatiques peuvent être diagnostiquées par la détection ou le dosage dans le sang de certaines molécules spécifiques (biomarqueurs). La cytolyse (nécrose des hépatocytes) est évaluée par la détection de protéines, contenues dans les cellules hépatiques, et qui sont déversées dans les voies sanguines suite à la nécrose des cellules hépatiques (biomarqueurs mécanistiques) : tel est le cas des transaminases qui reflètent une lyse des cellules du foie. Les transaminases ne sont cependant pas spécifiques du foie et leurs concentrations sanguines peuvent augmenter lors d'atteinte d'autres organes (cœur, muscle). Il peut également s'agir de molécules synthétisées par le foie, dont la fonction est impactée par la diminution de leur synthèse (biomarqueurs fonctionnels): c'est le cas du taux de prothrombine qui explore les facteurs de coagulation I, II, V, VII et X synthétisés par le foie. Cependant, du fait de la demi-vie sanguine de quelques heures des facteurs de coagulation, les troubles de la coagulation ne sont pas immédiats.

En ce qui concerne la stéatose hépatique non alcoolique et alcoolique, le diagnostic de certitude est habituellement réalisé sur une biopsie du foie du patient; ainsi on peut évaluer les lésions tissulaires hépatiques et déterminer la sévérité de la maladie. Par exemple, les transcrits des gènes *ADH1* et *ADH2* sont augmentés en RT-PCR dans un échantillon de biopsie de foie d'individus atteints de NAFLD ou de NASH et la protéine ADH1B est détectable par immunohistochimie dans un échantillon de biopsie de foie d'individus atteints de NAFLD (An Afida Ashla et al., Hepatology Research, 2010, 40, 594-604). Une biopsie est un examen invasif, associé à des risques hémorragiques, et nécessite une prise en charge hospitalière; c'est donc un examen coûteux. Il serait préférable de pouvoir détecter les troubles hépatiques et suivre leur évolution par des méthodes non invasives, de préférence à partir d'un liquide biologique, par exemple provenant d'une simple prise de sang.

Ces dernières années, un certain nombre de méthodes non invasives d'évaluation de lésions hépatiques ont été proposées, notamment pour détecter la stéatohépatite alcoolique et non alcoolique. A titre d'exemple, US 2006 / 0 172 286 décrit un procédé non invasif permettant le diagnostic de la stéatohépatite non alcoolique ou alcoolique chez un patient, comprenant la mesure des 3 marqueurs biochimiques ApoAI, ALT et AST dans un échantillon de sérum ou de plasma. WO 2010 / 058 295 décrit un procédé permettant de quantifier les lésions du foie d'un patient par l'intermédiaire du dosage d'un ou plusieurs biomarqueur (dont ALT). Zhiyuan Hu et al. (Theranostics, 2014, 4, 215-228) et Beger et al. (Arch. Toxicol., 2015, 89, 1497-1552) décrivent des biomarqueurs des lésions hépatiques aiguës induites par le paracétamol incluant le marqueur conventionnel transaminase (ALT1), ainsi que d'autres marqueurs tels que BHMT, notamment BHMT1. WO 2015 / 071 669 décrit une méthode de diagnostic, de pronostic ou de surveillance d'une tumeur du foie chez un individu par l'intermédiaire de la détection ou la quantification d'un ou plusieurs marqueurs présents notamment dans le sang ; la liste des marqueurs est extrêmement vaste et comprend notamment les protéines ADH1A, ADH1B, GSTA1, FABP1, ARG1, GPT1 (correspondant à l'ALT1), BHMT et ADH4.

En tout état de cause, la quantification absolue, c'est-à-dire la détermination de la concentration de protéines ou peptides présents dans un échantillon liquide, notamment un échantillon sanguin peut être un procédé assez complexe.

On constate que diagnostiquer des atteintes hépatiques à un stade précoce et prédire l'évolution de ces atteintes est devenu un enjeu de santé majeur, en particulier pour la stéatohépatite non alcoolique dont la prévalence augmente avec l'obésité.

La présente invention cherche à proposer un procédé de de diagnostic *in vitro* d'atteintes hépatiques, plus particulièrement un nouveau test de diagnostic non invasif de troubles hépatiques plus précis que les procédés de de diagnostic de l'art antérieur.

### Objets de l'invention

La présente invention est telle que définie dans les revendications. La présente invention concerne un procédé de diagnostic et/ou de suivi et/ou de pronostic et/ou théranostique *in vitro* de troubles hépatiques choisis parmi l'insuffisance hépatique aigüe, la stéatose hépatique et la stéatohépatite, à partir d'un échantillon sanguin issu d'un individu, dans lequel procédé on détermine la présence et/ou la concentration du marqueur ADH1B (SEQ ID N° 2) et/ou la présence et/ou la concentration de la combinaison des marqueurs ADH1B (SEQ ID N° 2) et ADH1A (SEQ ID N° 1).

Dans un mode de réalisation du procédé ci-dessus, on détermine la présence et/ou la concentration d'au moins un marqueur supplémentaire, préférentiellement d'au moins deux marqueurs supplémentaires, et encore plus préférentiellement d'au moins trois marqueurs supplémentaires, lesdits marqueurs étant sélectionnés dans le groupe constitué par : ADH4 (SEQ ID N° 3 & SEQ ID N° 4), BHMT (SEQ ID N° 5, SEQ ID N° 6 & SEQ ID N° 7), ARG1 (SEQ ID N° 8, SEQ ID N° 9 & SEQ ID N° 10), FABP1 (SEQ ID N° 11), GSTA1 (SEQ ID N° 12) et ALT1 (SEQ ID N° 13).

Dans un mode de réalisation du procédé ci-dessus, on détermine au moins la présence et/ou la concentration d'au moins un marqueur supplémentaire sélectionné parmi ADH4 (SEQ ID N° 3 & SEQ ID N° 4) et BHMT (SEQ ID N° 5, SEQ ID N° 6 & SEQ ID N° 7).

Dans un mode de réalisation du procédé ci-dessus, on détermine au moins la présence et/ou la concentration du marqueur ADH1B (SEQ ID N° 2) ou de la combinaison des marqueurs ADH1B (SEQ ID N° 2) et ADH1A (SEQ ID N° 1), d'une part, et des marqueurs ADH4 (SEQ ID N° 3 & SEQ ID N° 4) et BHMT (SEQ ID N° 5, SEQ ID N° 6 & SEQ ID N° 7), d'autre part.

Dans un mode de réalisation avantageux du procédé ci-dessus, le marqueur BHMT est le variant de séquence BHMT1 (SEQ ID N° 5) et/ou la combinaison des variants de séquence BHMT1 (SEQ ID N° 5) et BHMT2 (SEQ ID N° 6 & SEQ ID N° 7).

Dans un mode de réalisation du procédé décrit ci-dessus, on détermine en outre la présence et/ou la concentration du marqueur CK18.

Dans un mode de réalisation du procédé décrit ci-dessus, on utilise pour ladite détermination de la présence et/ou de la concentration desdits marqueurs des peptides signatures présents dans lesdits marqueurs.

Dans un mode de réalisation avantageux du procédé décrit ci-dessus, lesdits peptides signatures sont générés à partir desdits marqueurs par un procédé de digestion, de préférence enzymatique.

Dans un mode de réalisation du procédé décrit ci-dessus, la présence et/ou la concentration :
a. de la combinaison des marqueurs ADH1A (SEQ ID N° 1) et ADH1B (SEQ ID N° 2), si elle est sélectionnée, est déterminée par la présence d'au moins un peptide signature choisi parmi INEGFDLLHSGK (SEQ ID N° 16) et FSLDALITHVLPFEK (SEQ ID N° 15),
b. du marqueur ADH1B (SEQ ID N° 2), s'il est sélectionné, est déterminée par la présence du peptide signature AAVLWEVK (SEQ ID N° 17),
c. du marqueur ADH4 (SEQ ID N° 3 & SEQ ID N° 4), s'il est sélectionné, est déterminée par la présence d'au moins un peptide signature choisi parmi FNLDALVTHTLPFDK (SEQ ID N° 20), IDDDANLER (SEQ ID N° 18) ou IIGIDINSEK (SEQ ID N° 19),
d. du marqueur ARG1 (SEQ ID N° 8, SEQ ID N° 9 & SEQ ID N° 10), s'il est sélectionné, est déterminée par la présence d'au moins un peptide signature choisi parmi DVDPGEHYILK (SEQ ID N° 25), TIGIIGAPFSK (SEQ ID N° 24) ou EGLYITEEIYK (SEQ ID N° 26),
e. du marqueur BHMT (SEQ ID N° 5, SEQ ID N° 6 & SEQ ID N° 7), s'il est sélectionné, est déterminée par la présence d'au moins un peptide signature choisi parmi EATTEQQLK (SEQ ID N° 21), AIAEELAPER (SEQ ID N° 23) ou EAYNLGVR (SEQ ID N° 22),
f. du marqueur BHMT1 (SEQ ID N° 5), s'il est sélectionné, est déterminée par la présence du peptide signature EATTEQQLK (SEQ ID N° 21),
g. de la combinaison des marqueurs BHMT1 (SEQ ID N°5) et BHMT2 (SEQ ID N° 6 & SEQ ID N° 7), si elle est sélectionnée, est déterminée par la présence d'au moins un peptide signature choisi parmi AIAEELAPER (SEQ ID N° 23) ou EAYNLGVR (SEQ ID N° 22),
h. du marqueur FABP1 (SEQ ID N° 11), s'il est sélectionné, est déterminée par la présence d'au moins un peptide signature choisi parmi AIGLPEELIQK (SEQ ID N° 27), FTITAGSK (SEQ ID N° 28) ou TVVQLEGDNK (SEQ ID N° 29),
i. du marqueur GSTA1 (SEQ ID N° 12), s'il est sélectionné, est déterminée par la présence du peptide signature LHYFNAR (SEQ ID N°30),
j. du marqueur ALT1 (SEQ ID N° 13), s'il est sélectionné, est déterminée par la présence d'au moins un peptide signature choisi parmi ALELEQELR (SEQ ID N° 34), LLVAGEGHTR (SEQ ID N° 35) ou KPFTEVIR (SEQ ID N° 36), sachant que ALT1 (SEQ ID N° 13), s'il est sélectionné, est plus particulièrement déterminée par la présence du peptide signature ALELEQELR (SEQ ID N° 34) ou LLVAGEGHTR (SEQ ID N° 35).

Dans un mode de réalisation avantageux du procédé décrit ci-dessus, le procédé dans lequel on dose au moins un desdits marqueurs (dit « marqueur sélectionné »), dont au moins un marqueur ADH1B (SEQ ID N° 2) et/ou la combinaison des marqueurs ADH1B (SEQ ID N° 2) et ADH1A (SEQ ID N° 1), à partir d'un échantillon sanguin prélevé sur un individu à un temps t₀, par l'intermédiaire d'au moins un peptide signature caractéristique dudit marqueur, comprend les étapes suivantes :
a) l'ajout d'une quantité connue d'une protéine isotopiquement marquée (appelée « standard ») homologue du marqueur sélectionné dans l'échantillon sanguin
b) le traitement de l'échantillon sanguin pour extraire au moins une partie des protéines abondantes ou au moins une partie des glycoprotéines,
c) le traitement de l'échantillon après extraction d'au moins une partie des protéines abondantes ou des glycoprotéines pour générer des peptides protéolytiques, de préférence par digestion, étant entendu que parmi les peptides protéolytiques obtenus se trouvent les peptides signatures des marqueurs sélectionnés,
d) le dosage quantitatif par spectrométrie de masse d'au moins un peptide signature généré à partir du marqueur sélectionné,
e) la détermination d'un rapport de l'abondance du peptide signature isotopiquement marqué, issu du standard, ajouté à l'étape a), sur l'abondance du peptide signature non marqué issu de l'échantillonsanguin,
f) le calcul à partir du rapport obtenu à l'étape e) et de la quantité connue de ladite protéine isotopiquement marquée ajoutée à l'étape a), de la concentration dudit marqueur sélectionné dans l'échantillon.

Dans un mode de réalisation avantageux, l'étape b) du procédé ou de l'utilisation décrite ci-dessus, est effectuée par déplétion et préférentiellement par glycodéplétion.

Dans un mode de réalisation avantageux, l'étape b) du procédé ou de l'utilisation décrite ci-dessus, est suivie d'une étape d'électrophorèse de l'échantillon sanguin.

Dans un mode de réalisation avantageux, le procédé est effectué à un temps t₀ et à un temps t₁ supérieur à t₀, et comprend après l'étape f), la comparaison de la concentration du marqueur sélectionné dans l'échantillon entre t₁ et t₀.

Dans un mode de réalisation avantageux, l'étape c) du procédé décrit ci-dessus est effectuée par digestion enzymatique, de préférence par l'emploi de trypsine ou d'un mélange d'EndolysC et de trypsine.

Le trouble hépatique est une insuffisance hépatique aiguë, une stéatose hépatique ou une stéatohépatite, de préférence une insuffisance hépatique aiguë ou une stéatohépatite non-alcoolique. L'hépatite aigüe est notamment une hépatite virale, une hépatite alcoolique, une hépatite médicamenteuse, une hépatite liée à une intoxication, par exemple aux champignons ou aux vitamines, une hépatite auto-immune ou la maladie de Wilson ; de préférence il s'agit d'une hépatite non-alcoolique, telle que notamment une hépatite médicamenteuse, par exemple due au paracétamol ou un autre médicament. La stéatose hépatique est en particulier une stéatose non-alcoolique, telle qu'une stéatose isolée (NAFL pour « Non-Alcoholic Fat Liver »). La stéatohépatite est en particulier une stéatohépatite non-alcoolique (NASH pour « Non-Alcoholic SteatoHepatitis »).Un autre objet de l'invention concerne une utilisation du procédé pour évaluer la toxicité de molécules (candidats médicaments, médicament, xénobiotique) préalablement administrées à l'individu dont est issu ledit échantillon sanguin.

### Description des figures

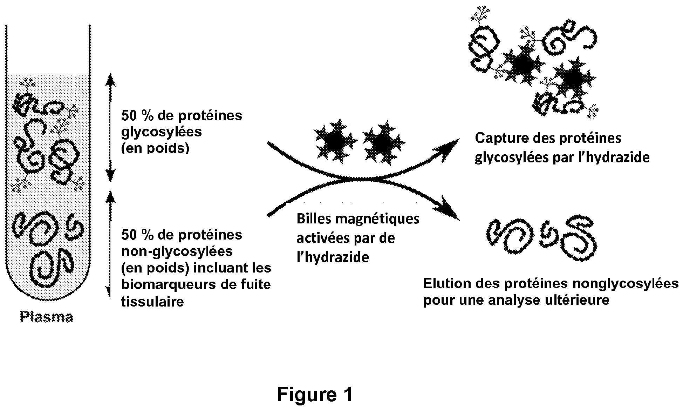

Les figures 1 à 8 illustrent certains aspects de l'invention.
La figure 1 décrit le principe de la glycodépletion de protéines plasmatiques pour le dosage de biomarqueurs non glycosylés (comme les protéines de lyse hépatocytaire).
   Le protéome plasmatique est composé (en poids) de 50% des protéines glycosylées et de 50% de protéines non glycosylées. Les protéines glycosylées peuvent être capturées sur des billes magnétiques fonctionnalisées par de l'hydrazide via la formation de liaisons covalentes. Les protéines non glycosylées sont ensuite éluées avec de l'urée permettant de casser les interactions protéines / protéines.

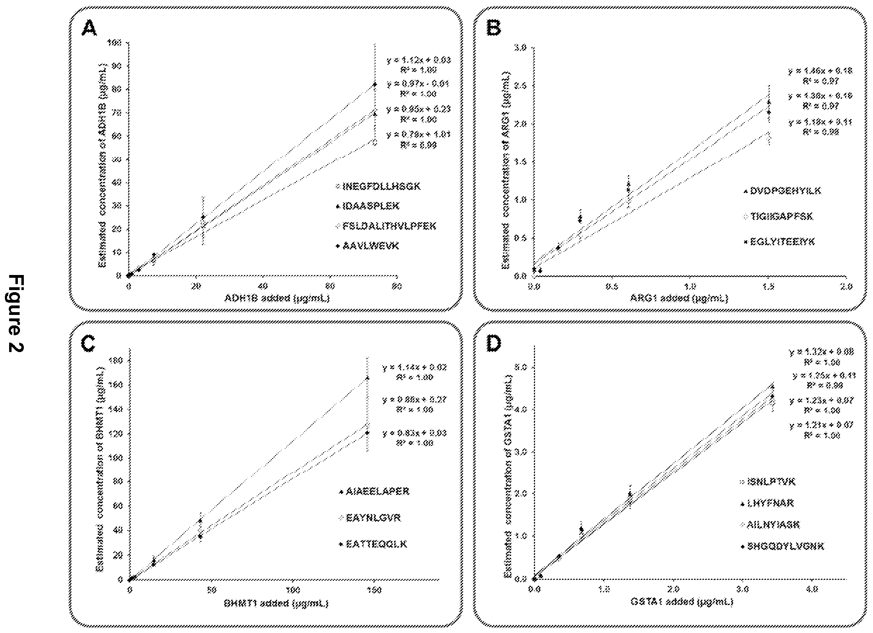

La figure 2 présente les courbes d'étalonnage obtenues pour ADH1B, ARG1, BHMT1 et GSTA1 dans le plasma après immunodéplétion. Ces courbes d'étalonnage permettent d'évaluer les performances analytiques de la méthode de dosage.

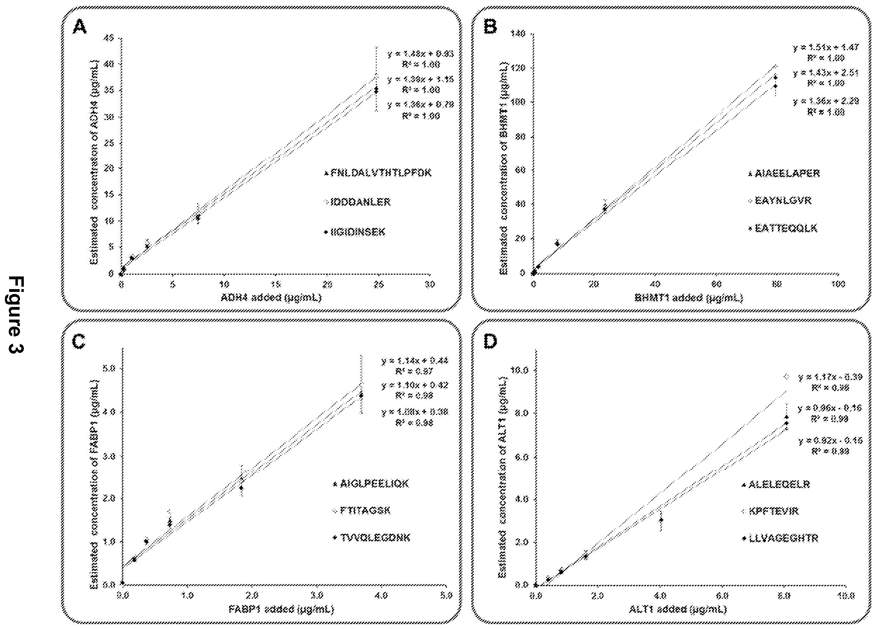

La figure 3 présente les courbes d'étalonnage obtenues pour ADH4, BHMT1, FABP1 et ALT1 dans le plasma après glycodéplétion.

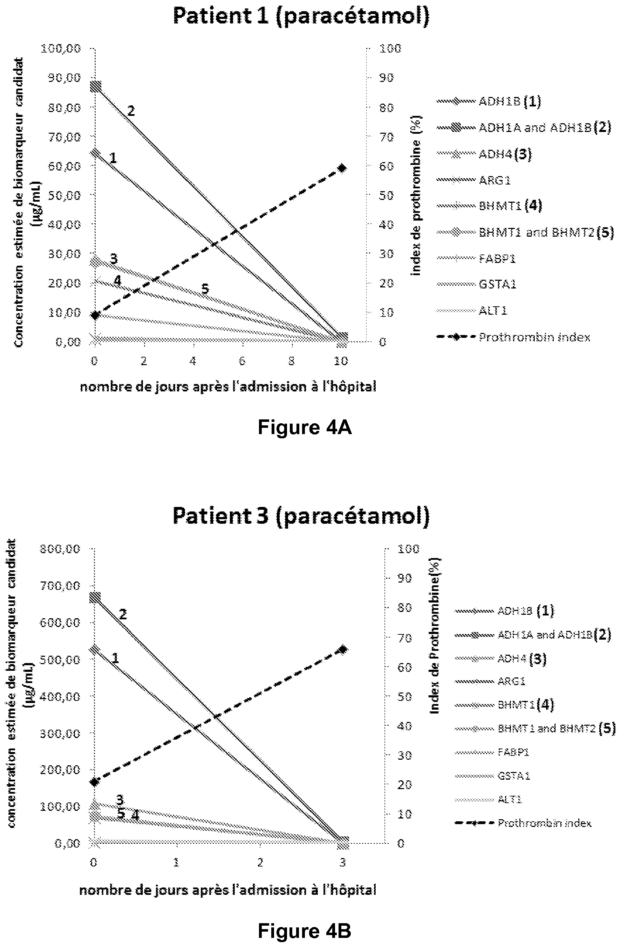

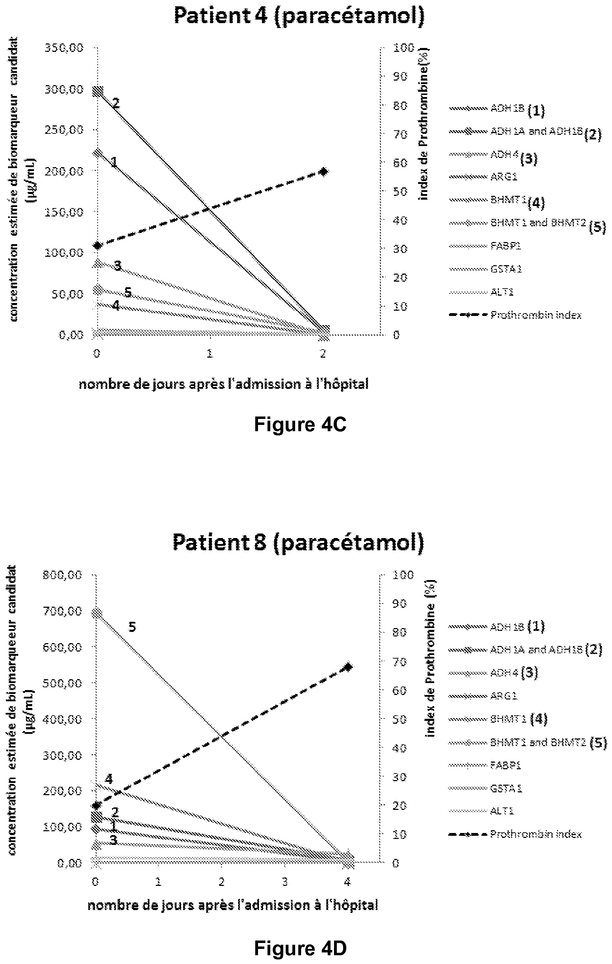

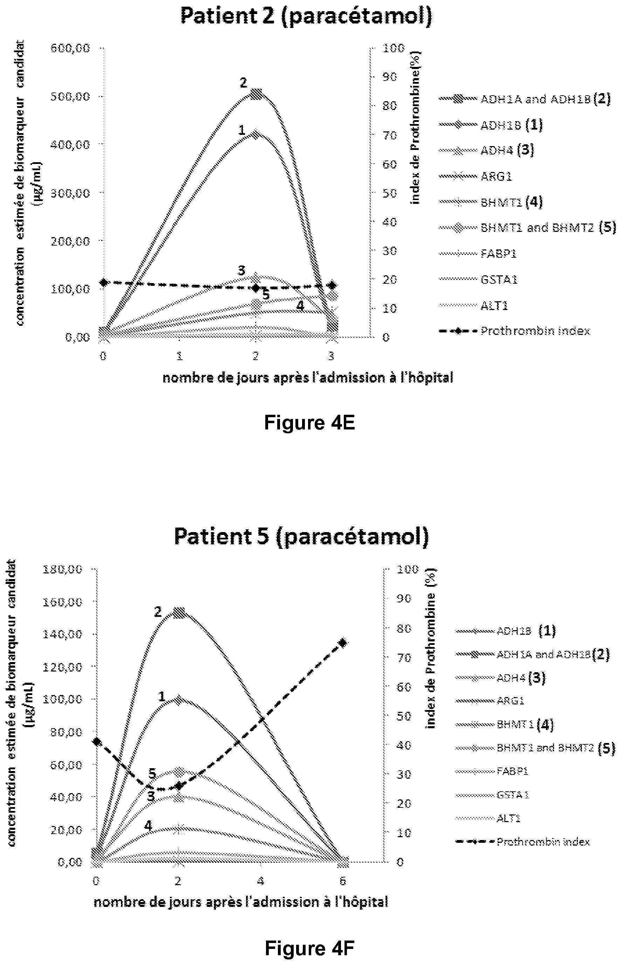

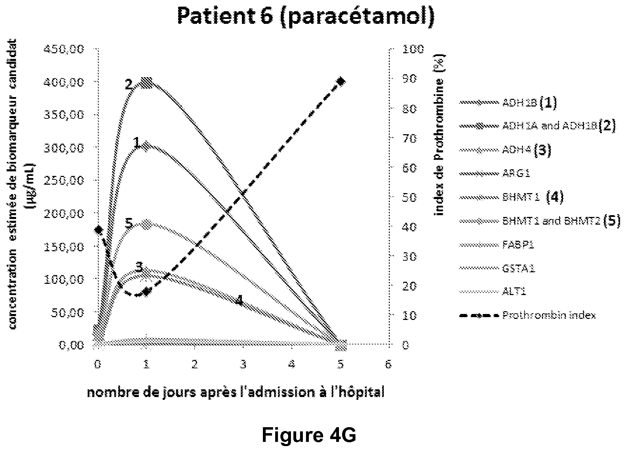

Les figures 4 (A à G) décrivent l'analyse des biomarqueurs dans des échantillons sanguins issus de patients ayant une insuffisance hépatique induite par du paracétamol. Les concentrations des biomarqueurs cibles ont été déterminées en utilisant les standards PSAQ™ après préfractionnement de sérum par LC-SRM. Le taux de prothrombine est mentionné comme un indicateur de troubles de la coagulation et d'une insuffisance hépatique ALI ou ALF.

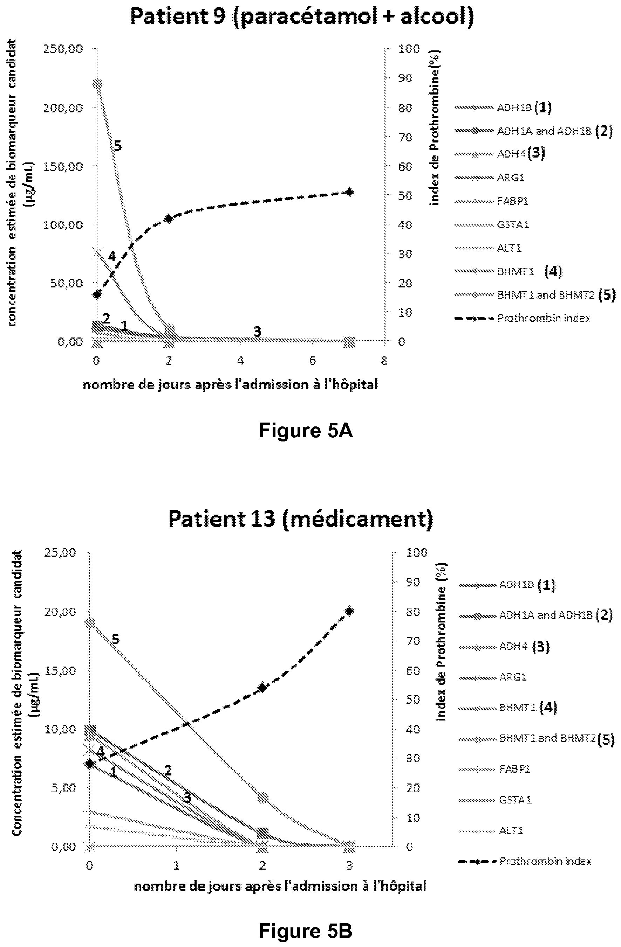

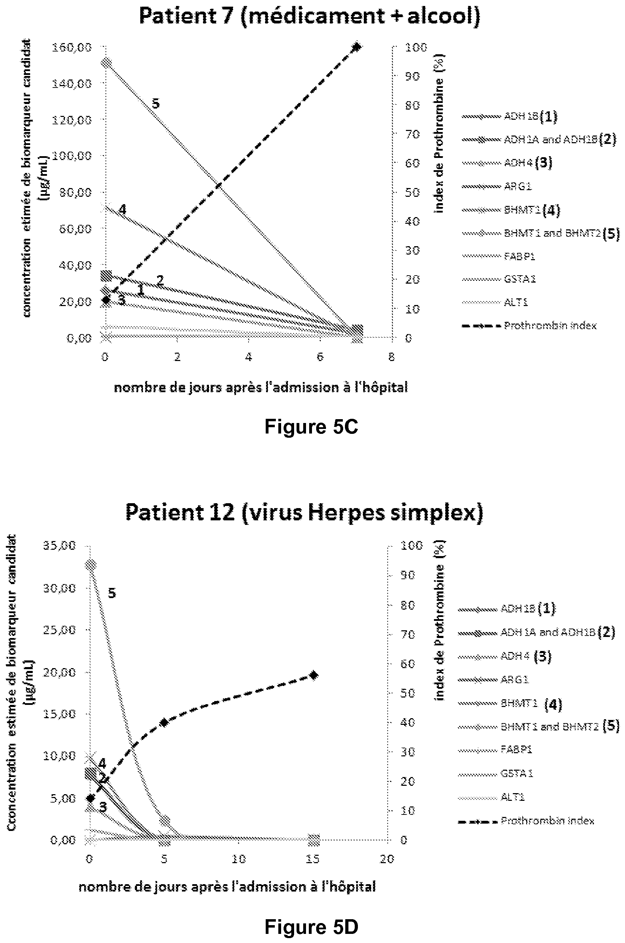

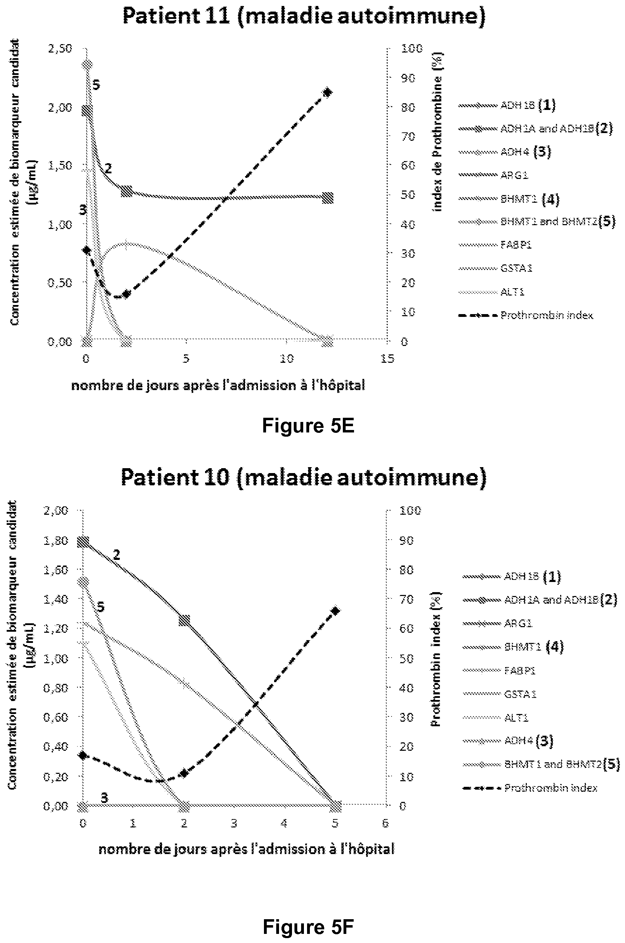

Les figures 5 (A à F) décrivent l'analyse des biomarqueurs dans des échantillons sanguinss issus de patients ayant une atteinte hépatique ALI ou ALF induite par une cause autre que le paracétamol. Les concentrations des biomarqueurs cibles ont été déterminées en utilisant les standards PSAQ™ (WO 2008 / 145 763 A1) après préfractionnement de sérum et analyse LC-SRM. Le taux de prothrombine est mentionné comme un indicateur de troubles de la coagulation et d'une insuffisance hépatique ALI/ALF.

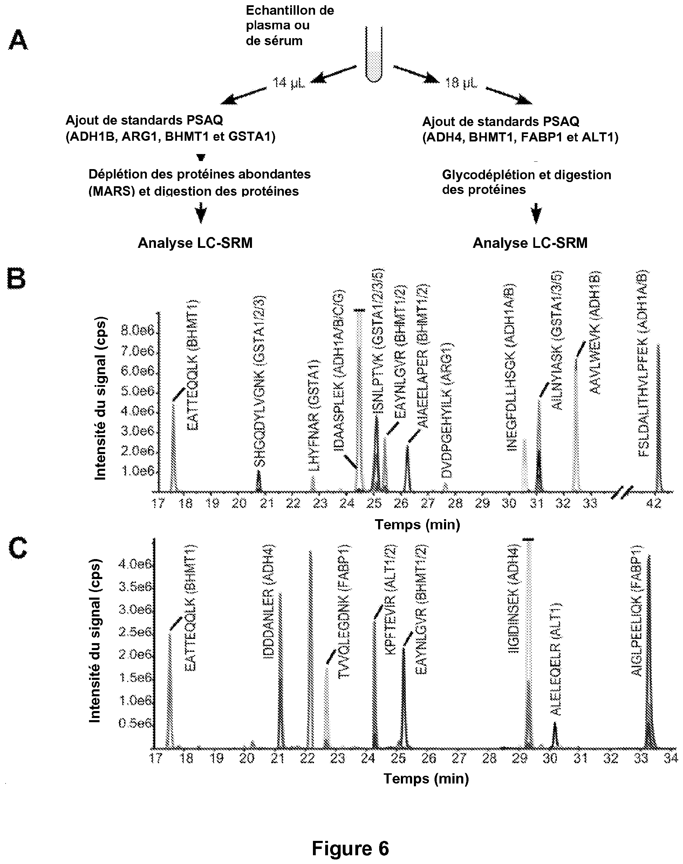

La figure 6 décrit la méthode de standardisation, préparation et analyse des échantillons. Le panel A correspond à la procédure de standardisation et de préparation. Les panels B et C correspondent aux signaux des différents peptides signatures obtenus par analyse LC-SRM.

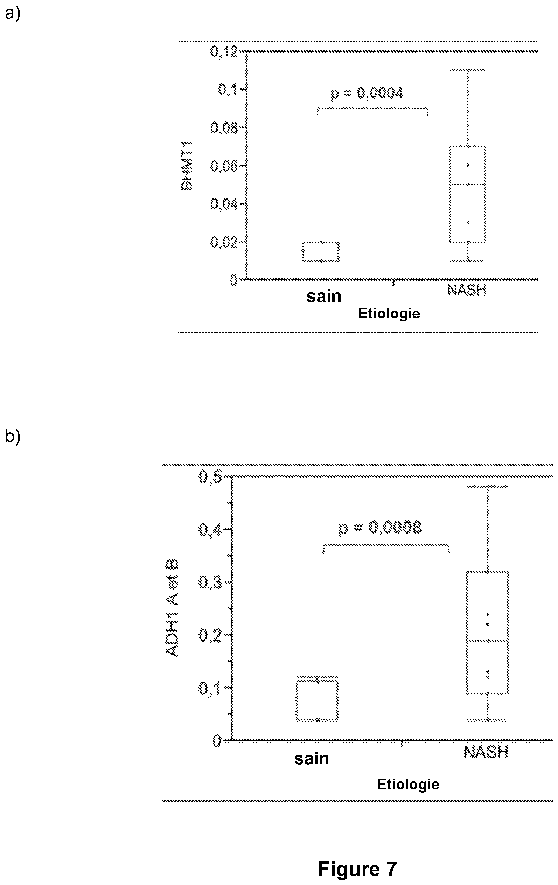

La figure 7 montre les concentrations plasmatiques de BHMT1 (cf. Figure 7a) et les concentrations plasmatiques des variants A et B de l'ADH1 (cf. Figure 7b) chez des patients atteints de stéatohépatites non alcooliques (NASH) en comparaison de donneurs sains appariés par âge et sexe (n = 11 individus dans chaque groupe). Les concentrations plasmatiques sont mentionnées en ordonnée en µg/mL. Un test statistique de Mann-Whitney a été appliqué avec p < 0.05 comme seuil de significativité.

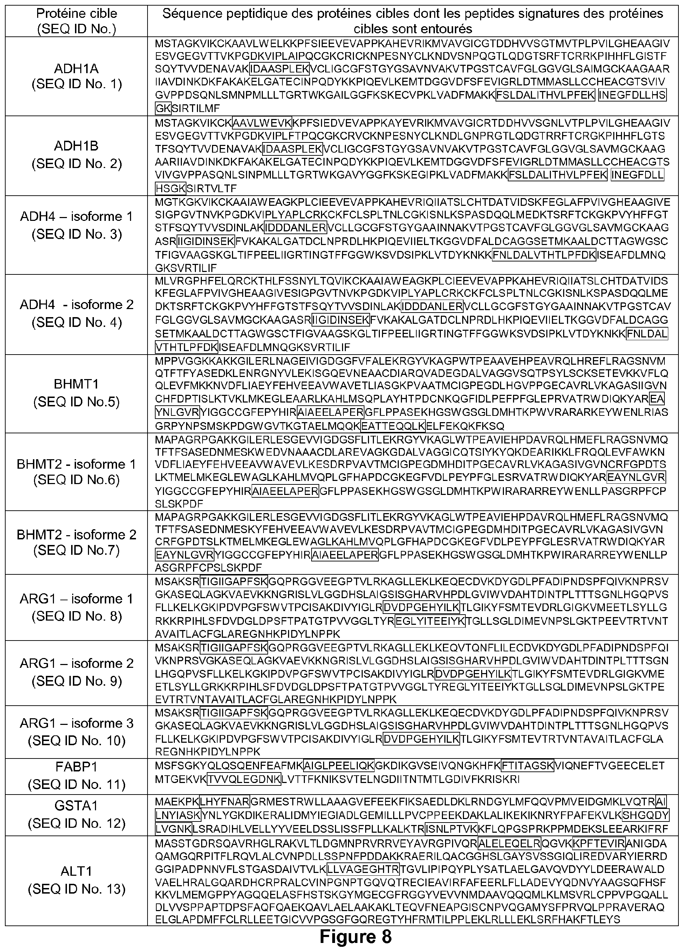

La figure 8 présente les séquences peptidiques des protéines ADH1A (SEQ ID N° 1), ADH1B (SEQ ID N° 2), ADH4 (SEQ ID N° 3 & SEQ ID N° 4), BHMT1 (SEQ ID N°5), BHMT2 (SEQ ID N° 6 & SEQ ID N° 7), ARG1 (SEQ ID N° 8, SEQ ID N° 9 & SEQ ID N° 10), FABP1 (SEQ ID N° 11), GSTA1 (SEQ ID N° 12) et ALT1 (SEQ ID N° 13). Les peptides analysés de ces protéines cibles sont visualisés dans la séquence peptidique des protéines par un encadrement. Ces peptides sont des peptides issus de la digestion des protéines cibles et servent de substituts pour l'analyse de ces protéines en spectrométrie de masse.

### Description détaillée

Dans la présente invention, on entend par « atteintes hépatiques » ou « troubles hépatiques » ou « lésions hépatiques », les hépatites virales, les hépatites alcooliques, les hépatites médicamenteuses, les hépatites liées à des intoxications tels que celles dues aux champignons ou aux vitamines, les hépatites auto-immunes, la stéatose hépatique (NAFLD), les stéatohépatites (NASH), les cirrhoses et la maladie de Wilson à l'exclusion des hépatocarcinomes.

Dans le cadre de la présente invention, les protéines peuvent être dosées par n'importe quelle technique classiquement utilisée par l'homme de l'art comme des méthodes de dosages immunologiques utilisant des anticorps spécifiques ou des méthodes basées sur la spectrométrie de masse. Parmi les méthodes de dosage basées sur la spectrométrie de masse, on peut citer la technologie AQUA (Gerber et al, « Absolute quantification of proteins and phosphoproteins from cell lysates by tandem MS » Proc Natl Acad Sci U S A. (2003) 100(12):6940-6945.) ou la technologie PSAQ™ (Protein Standard Absolute Quantification) décrite dans la demande WO 2008 / 145 763.

La méthode selon l'invention est basée sur la détection et le dosage de certains biomarqueurs issus de la lyse des cellules hépatiques ; ces biomarqueurs sont des protéines. Selon un mode de réalisation préféré de l'invention, le dosage se fait par spectrométrie de masse via la technologie PSAQ™. Selon cette méthode, la quantification absolue d'une protéine par spectrométrie de masse nécessite un étalon. Dans la présente invention, on entend par protéine PSAQ™ ou standard PSAQ™ ou protéine étalon toute protéine entière recombinante marquée isotopiquement, chimiquement analogue à la protéine native que l'on souhaite doser. Le principe de la méthode PSAQ™ selon l'invention repose sur l'introduction dans un échantillon sanguin d'une quantité connue d'une protéine-étalon entière recombinante marquée isotopiquement, identique ou analogue (i.e, structurellement proche, elle peut différer par exemple par l'addition d'une étiquette de purification) à la protéine native que l'on souhaite doser. Contrairement aux autres méthodes de quantification absolue, une quantité connue de la protéine-étalon est injectée directement dans l'échantillon sanguin avant tout prétraitement de cet échantillon. La méthode selon l'invention permet de déterminer, dans un échantillon sanguin tel que le plasma et le sérum, les concentrations de certaines protéines cibles dont ils constituent des analogues marqués.

Le procédé selon l'invention est avantageusement effectué sur un échantillon biologique issu d'un prélèvement de sang sur un individu.

Le procédé selon l'invention peut notamment être effectué sur le sang, le plasma et/ou le sérum.

### Standards PSAQ™ - Protéines-étalons

De nombreuses protéines contribuent aux fonctions métaboliques du foie. Selon l'invention les protéines permettant de caractériser des lésions du foie sont choisies en fonction de leur expression hépatique prédominante et de leur forte détectabilité dans le plasma, à savoir l'ADH1A et l'ADH1B, l'ADH4, l'ARG1, la BHMT (variants de séquence 1 et 2), la FABP1, la GSTA1 et l'ALT1.

Les alcools déshydrogénases (ADH) sont des oxydoréductases qui participent à la détoxication de l'organisme par l'élimination des alcools toxiques. Plusieurs types d'ADH existent (variants ou isoformes). Concernant l'ADH1, il en existe 3 variants : l'ADH1A, l'ADH1B et l'ADH1C. Ces variants ont été étudiés au niveau génétique. Duell et al. ont mis en évidence l'influence de certaines mutations génétiques de l'ADH, notamment sur la sensibilité à l'alcoolisme et les risques de cancer gastrique des individus porteurs de ces mutations génétiques *(*« Genetic variation in alcohol dehydrogenase (ADH1A, ADH1B, ADH1C, ADH7) and aldehyde dehydrogenase (ALDH2), alcohol consumption and gastric cancer risk in the european prospective investigation into cancer and nutrition (EPIc) cohort », Carcinogenesis vol.33, n°2, p361-367 (2012)).

L'ADH1A (SEQ ID N° 1) et l'ADH1B (SEQ ID N° 2) participent à l'oxydation de l'éthanol et jouent un rôle majeur dans le catabolisme de l'éthanol. L'ADH4 participe à l'oxydation des alcools aliphatiques et/ou des alcools aromatiques. L'ADH4 existe sous forme de 2 isoformes (SEQ ID N° 3 & SEQ ID N° 4). Dans l'étude, les inventeurs se sont intéressés à l'ADH1B mais aussi au mélange des variants ADH1A et ADH1B.

La BHMT (Betaine-homocystéine-methyltransférase) est une métallo-enzyme à base de zinc qui participe au métabolisme de l'homocystéine, de la glycine, de la sérine, de la thréonine et aussi de la méthionine. Par ce terme BHMT on entend les variants de séquences BHMT1 (SEQ ID N° 5), BHMT2, ainsi que les isoformes de BHMT2 (SEQ ID N° 6 & SEQ ID N° 7).

L'arginase ARG1 est une hydrolase ; cette métalloenzyme hépatique à base de manganèse intervient dans le cycle de l'urée. Par ce terme ARG1 on entend les trois isoformes de l'arginase 1 (SEQ ID N° 8, SEQ ID N° 9 & SEQ ID N° 10).

La FABP1 (« Fatty acid-binding protein, Liver » ; SEQ ID N° 11) est une protéine de liaison aux acides gras spécifiques du foie servant à transporter les acides gras des membranes cellulaires vers les mitochondries.

Les glutathione S-transférases (GSTA) sont des enzymes qui catalysent des reactions de conjugaison du glutathion réduit (GSH) avec des xénobiotiques afin de les détoxifier. Dans cette étude, les inventeurs ont étudié le variant GSTA1 (SEQ ID N° 12) mais aussi le pool de plusieurs variants en particulier le pool des variants GSTA1, GSTA2, GSTA3 et le pool des 4 variants GSTA1, GSTA2, GSTA3 et GSTA5.

L'alanine cytosolique aminotransaminase 1 ou ALT1 (SEQ ID N° 13), également connue sous le nom de glutamate-pyruvate transaminase 1, est une enzyme faisant partie des transaminases jouant un rôle essentiel dans le métabolisme intermédiaire du glucose. Cette protéine est connue en tant que biomarqueur de lésion hépatique provoquée par certains médicaments, de l'alcool, et par la stéatose.

Le tableau 1 résume certaines propriétés de ces protéines.

Les protéines ADH1A et ADH1B, ADH4, ARG1, BHMT (variants 1 et 2), FABP1, GSTA1 et ALT1 sont principalement exprimées dans le foie. Chez un individu sain, ces protéines sont principalement présentes dans le foie (hépatocytes) et dans quelques autres types cellulaires différents. C'est le cas, par exemple, de la protéine ADH1B qui est détectable dans uniquement trois types cellulaires différents parmi les 80 analysés (cf. tableau 1, atlas des protéines humaines). A titre comparatif et selon l'atlas des protéines humaines, dans un tissu sain (www.proteinatlas.org), l'ALT1 qui est un marqueur usuel de lésions hépatiques est exprimé dans 29 types cellulaires parmi les 78 analysés. Les protéines ADH1A et ADH1B, ADH4, ARG1, BHMT (variants 1 et 2), FABP1, GSTA1 sont donc bien plus spécifiques du foie que l'ALT1.

**Tableau 1 : Biomarqueurs sélectionnés dans le procédé selon l'invention pour le suivi d'atteintes hépatiques**

| Protéine (SEQ ID N°) | Identifiant Uniprot | Nombre de types cellulaires dans un tissu sain dans lequel la protéine est détectée (a) | Masse moléculaire [Da] | Fonction biochimique |
|---|---|---|---|---|
| ADH1B (SEQ ID N° 2) | P00325 | 3 sur 80 analysés | 39 855 | Métabolisme des xénobiotiques |
| ADH1A (SEQ ID N° 1) | P07327 | Hepatocytes | 39 859 | Métabolisme des xénobiotiques |
| ADH4 (SEQ ID N° 3 & SEQ ID N° 4) | P08319 | 6 sur 83 analysés | 40 222 | Métabolisme des xénobiotiques |
| ARG1 (SEQ ID N° 8, SEQ ID N° 9 & SEQ ID N° 10) | P05089 | 2 sur 83 analysés | 34 735 | Métabolisme de l'azote et cycle de l'urée |
| BHMT1 (SEQ ID N° 5) | Q93088 | 3 sur 83 analysés | 44 998 | Métabolisme de l'homocystéine |
| FABP1 (SEQ ID N° 11) | P07148 | 7 sur 83 analysés | 14 208 | Métabolisme des lipides et transport des lipides |
| GSTA1 (SEQ ID N° 12) | P08263 | 16 sur 81 analysés | 25 631 | Métabolisme des xénobiotiques et de la glutathione |

| | | | | |
|---|---|---|---|---|
| (a) Source: The Human Protein Atlas (www.proteinatlas.org). | | | | |

Selon l'invention, d'autres biomarqueurs tels que la protéine cytokeratine 18 (CK18) peuvent être employés en complément d'analyse afin de confirmer un trouble hépatique et d'établir un diagnostic. En effet, lorsque les hépatocytes sont exposés de façon chronique au stress oxydatif et à des substances toxiques, ils gonflent, accumulent de la graisse et montrent une perturbation dans le réseau de filaments de kératine, et forment des corps de Mallory, i.e. des amas résiduels de microfilaments. Un corps de Mallory est composé de kératines anormalement phosphorylées et réticulées, telles que la cytokératine CK18. On sait que les hépatocytes contenant des corps de Mallory sont sensibles à l'apoptose, induisant une libération importante des protéines CK18 et des corps de Mallory dans le sang. La publication de Maher et al. « Cytokeratin 18 as a non invasive marker in diagnosis of NASH and its usefulness in correlation with disease severity in Egyptian patients » publiée dans The Egyptian Journal of Medical Human Genetics (2015) 16, p.41 à 46, présente la CK18 comme un marqueur non-invasif permettant de corréler sa concentration au degré de sévérité de la NASH. Effectivement, lorsque la protéine CK18 est libérée dans le sang sous forme entière (forme M65), elle indique un phénomène de nécrose. Quand elle est libérée sous forme tronquée (forme M30) elle indique un phénomène d'apoptose. Généralement les concentrations sanguines de CK18 sont mesurées sous ces 2 formes et le ratio entre ces deux formes est calculé. La CK18 n'est pas exprimée de manière spécifique dans les hépatocytes, elle est présente dans les épithéliums glandulaires.

### Rôle des biomarqueurs

Dans le sérum ou plasma de patients sains, les protéines ADH1 (variant B ou isoformes A et B) et ADH4 sont présentes à des concentrations inférieures à 2 µg/mL, l'ARG1 à des concentrations inférieures à 0.3 µg/mL, la FABP1 à des concentrations inférieures à 1 µg/mL, la GSTA1, GSTA2, GSTA2 et GSTA5 à des concentrations inférieures à 1 µg/mL, la BHMT (variants ou isoformes 1 et 2) à des concentrations inférieures à 2 µg/mL et l'ALT1 à des concentrations inférieures à 1 µg/mL (ces concentrations seuil ont été évaluées après analyse d'échantillons selon le protocole décrit dans la figure 6).

La Demanderesse s'est rendu compte que la concentration sérique ou plasmatique des protéines ADH1B, ADH1A et B, ADH4, ARG1, BHMT1 et BHMT1 et 2, FABP1, GSTA1 et ALT1 évolue en fonction de l'atteinte hépatique et de sa sévérité.

Selon les observations de la Demanderesse, la concentration des protéines ADH1B, ADH1A et ADH1B, ADH4, ARG1, FABP1, GSTA1, BHMT1 et BHMT (variants 1 et 2) augmente au cours des phases aiguës de l'insuffisance hépatique induite par des xénobiotiques tels que le paracétamol, puis chute à des niveaux très faibles au cours de la période de récupération / régénération du foie. Cette cinétique est étroitement corrélée avec celle des caractéristiques biologiques de cette maladie telles que le taux de prothrombine. Certaines de ces protéines sont plus exprimées au niveau hépatique que d'autres et sont donc plus facilement quantifiables ; c'est le cas des protéines ADH4, BHMT (variants 1 et 2), ADH1 (variants ADH1A et ADH1B) qui peuvent, être avantageusement employées, de manière individuelle ou combinée, comme biomarqueurs de diagnostic, de suivi et de pronostic de l'ALI ou l'ALF. La protéine ARG1 bien que détectée présente des modifications de sa concentration plasmatique ou sérique moins marquées mais corrélées avec le stade de l'atteinte (phase aigüe/phase de récupération).

Les marqueurs ADH1 (ADH1B seul ou combinaison d'ADH1B et ADH1A), ADH4 et BHMT (variant 1 seul ou combinaison des 2 variants 1 et 2) peuvent permettre une identification précoce de lésions hépatiques induites par des médicaments tels que le paracétamol, de manière à adapter la prise en charge médicale du patient (en particulier le recours à la greffe hépatique). De plus, contrairement aux marqueurs transaminases (dont ALT1) et CK18 dont les concentrations peuvent rester élevées, ces marqueurs ADH1 (ADH1B seul ou combinaison d'ADH1B et ADH1A), ADH4 et BHMT (variant 1 seul ou combinaison des 2 variants 1 et 2) présentent des taux sériques faibles au cours de la régénération spontanée du foie.

Pour ce qui est des troubles hépatiques non induits par des médicaments une modification des concentrations plasmatiques/sériques des biomarqueurs, notamment des protéines ADH1 (ADH1B et/ou ADH1B et ADH1A), ADH4 et BHMT (variant 1 seul ou combinaison des 2 variants 1 et 2) est observée à des niveaux plus faibles qu'avec le paracétamol mais elle est cohérente avec le statut clinique du patient.

Selon un mode de réalisation de l'invention, les marqueurs ADH1 (ADH1B et/ou (ADH1B et ADH1A)), ADH4, ARG1, FABP1, GSTA1, ALT1 et BHMT (variant 1 et/ou le mélange des deux variants 1 et 2) peuvent être employés pour la détection d'une lésion hépatique.

La détection d'une lésion hépatique peut être mise en évidence par la détection et/ou la quantification du marqueur ADH1, de préférence le marqueur ADH1B et/ou le pool d'ADH1A et ADH1B.

La détection d'une lésion hépatique peut être mise en évidence par la détection et/ou la quantification du marqueur ADH1 ((ADH1B et/ou (ADH1B et ADH1A)) et d'au moins un marqueur supplémentaire, d'au moins deux marqueurs supplémentaires, d'au moins trois marqueurs supplémentaires, d'au moins quatre marqueurs supplémentaires, d'au moins cinq marqueurs supplémentaires ou d'au moins six marqueurs supplémentaires choisis parmi : ADH4, BHMT (variant 1 et/ou combinaison des deux variants 1 et 2), ARG1, FABP1, GSTA1 et ALT1.

La détection d'une lésion hépatique peut être mise en évidence par la détection et/ou la quantification de deux biomarqueurs spécifiquement, tels que ADH1 ((ADH1B et/ou (ADH1B et ADH1A)) et ADH4 ; ADH1 (ADH1B et/ou (ADH1B et ADH1A)) et BHMT (variant 1 et/ou un mélange des deux variants 1 et 2) ; ADH1 (ADH1B et/ou (ADH1B et ADH1A)) et ARG1 ; ADH1 (ADH1B et/ou (ADH1B et ADH1A)) et FABP1 ; ADH1 (ADH1B et/ou (ADH1B et ADH1A)) et GSTA1 ou des 2 biomarqueurs ADH1 (ADH1B et/ou (ADH1B et ADH1A)) et ALT1.

La détection d'une lésion hépatique peut être mise en évidence par la détection et/ou la quantification de trois biomarqueurs spécifiquement, tels que ADH1 (ADH1B et/ou (ADH1B et ADH1A)), ADH4 et BHMT (variant 1 et/ou un mélange des deux variants 1 et 2); ADH1 (ADH1B et/ou (ADH1B et ADH1A)), ADH4 et ARG1 ; ADH1 (ADH1B et/ou (ADH1B et ADH1A)), ADH4 et FABP1 ; ADH1 (ADH1B et/ou (ADH1B et ADH1A)), ADH4 et GSTA1 ; ADH1 (ADH1B et/ou (ADH1B et ADH1A)), ADH4 et ALT1 ; ADH1 (ADH1B et/ou (ADH1B et ADH1A)), BHMT (variant 1 et/ou un mélange des deux variants 1 et 2) et ARG1 ; ADH1 (ADH1B et/ou (ADH1B et ADH1A)), BHMT (variant 1 et/ou un mélange des deux variants 1 et 2) et FABP1 ; ADH1 (ADH1B et/ou (ADH1B et ADH1A)), BHMT (variant 1 et/ou un mélange des deux variants 1 et 2) et GSTA1 ; ADH1 (ADH1B et/ou (ADH1B et ADH1A)), BHMT (variant 1 et/ou un mélange des deux variants 1 et 2) et ALT1 ; ADH1 (ADH1B et/ou (ADH1B et ADH1A)), ARG1 et FABP1 ; ADH1 (ADH1B et/ou (ADH1B et ADH1A)), ARG1 et GSTA1 ; ADH1 (ADH1B et/ou (ADH1B et ADH1A)), ARG1 et ALT1 ; ADH1 (ADH1B et/ou (ADH1B et ADH1A)), FABP1 et GSTA1 ; ADH1 (ADH1B et/ou (ADH1B et ADH1A)), FABP1 et ALT1 ou des 3 biomarqueurs ADH1 (ADH1B et/ou (ADH1B et ADH1A)), GSTA1 et ALT1.

La détection d'une lésion hépatique peut être mise en évidence par la détection et/ou la quantification de quatre biomarqueurs spécifiquement, tels que ADH1 (ADH1B et/ou (ADH1B et ADH1A)), ADH4, BHMT (variant 1 et/ou un mélange des deux variants 1 et 2) et ARG1 ; ADH1 (ADH1B et/ou (ADH1B et ADH1A)), ADH4, BHMT (variant 1 et/ou un mélange des deux variants 1 et 2) et FABP1 ; ADH1 (ADH1B et/ou (ADH1B et ADH1A)), ADH4, BHMT (variant 1 et/ou un mélange des deux variants 1 et 2) et GSTA1 ; ADH1 (ADH1B et/ou (ADH1B et ADH1A)), ADH4, BHMT (variant 1 et/ou un mélange des deux variants 1 et 2) et ALT1 ; ADH1 (ADH1B et/ou (ADH1B et ADH1A)), ADH4, ARG1 et FABP1 ; ADH1 (ADH1B et/ou (ADH1B et ADH1A)), ADH4, ARG1 et GSTA1 ; ADH1 (ADH1B et/ou (ADH1B et ADH1A)), ADH4, ARG1 et ALT1 ; ADH1 (ADH1B et/ou (ADH1B et ADH1A)), ADH4, FABP1 et GSTA1 ; ADH1 (ADH1B et/ou (ADH1B et ADH1A)), ADH4, FABP1 et ALT1 ; ADH1 (ADH1B et/ou (ADH1B et ADH1A)), ADH4, GSTA1 et ALT1 ; ADH1 (ADH1B et/ou (ADH1B et ADH1A)), BHMT (variant 1 et/ou un mélange des deux variants 1 et 2), ARG1 et FABP1 ; ADH1 (ADH1B et/ou (ADH1B et ADH1A)), BHMT (variant 1 et/ou un mélange des deux variants 1 et 2), ARG1 et GSTA1 ; ADH1 (ADH1B et/ou (ADH1B et ADH1A)), BHMT (variant 1 et/ou un mélange des deux variants 1 et 2), ARG1 et ALT1 ; ADH1 (ADH1B et/ou (ADH1B et ADH1A)), BHMT (variant 1 et/ou un mélange des deux variants 1 et 2), FABP1 et GSTA1 ; ADH1 (ADH1B et/ou (ADH1B et ADH1A)), BHMT (variant 1 et/ou un mélange des deux variants 1 et 2), FABP1 et ALT1 ; ADH1 (ADH1B et/ou (ADH1B et ADH1A)), BHMT (variant 1 et/ou un mélange des deux variants 1 et 2), GSTA1 et ALT1 ; ADH1 (ADH1B et/ou (ADH1B et ADH1A)), ARG1, FABP1 et GSTA1 ; ADH1 (ADH1B et/ou (ADH1B et ADH1A)), ARG1, FABP1 et ALT1 ; ADH1 (ADH1B et/ou (ADH1B et ADH1A)), ARG1, GSTA1 et ALT1 ou des quatre marqueurs ADH1 (ADH1B et/ou (ADH1B et ADH1A)), FABP1, GSTA1 et ALT1.

La détection d'une lésion hépatique peut être mise en évidence par la détection et/ou la quantification de cinq biomarqueurs spécifiquement, tels que ADH1 (ADH1B et/ou (ADH1B et ADH1A)), ADH4, BHMT (variant 1 et/ou un mélange des deux variants 1 et 2), ARG1 et FABP1 ; ADH1 (ADH1B et/ou (ADH1B et ADH1A)), ADH4, BHMT (variant 1 et/ou un mélange des deux variants 1 et 2), ARG1 et GSTA1 ; ADH1 (ADH1B et/ou (ADH1B et ADH1A)), ADH4, BHMT (variant 1 et/ou un mélange des deux variants 1 et 2), ARG1 et ALT1 ; ADH1 (ADH1B et/ou (ADH1B et ADH1A)), ADH4, BHMT (variant 1 et/ou un mélange des deux variants 1 et 2), FABP1 et GSTA1 ; ADH1 (ADH1B et/ou (ADH1B et ADH1A)), ADH4, BHMT (variant 1 et/ou un mélange des deux variants 1 et 2), FABP1 et ALT1 ; ADH1 (ADH1B et/ou (ADH1B et ADH1A)), ADH4, BHMT (variant 1 et/ou un mélange des deux variants 1 et 2), GSTA1 et ALT1 ; ADH1 (ADH1B et/ou (ADH1B et ADH1A)), ADH4, ARG1, FABP1 et GSTA1 ; ADH1 (ADH1B et/ou (ADH1B et ADH1A)), ADH4, ARG1, FABP1 et ALT1 ; ADH1 (ADH1B et/ou (ADH1B et ADH1A)), ADH4, ARG1, GSTA1 et ALT1 ; ADH1 (ADH1B et/ou (ADH1B et ADH1A)), ADH4, FABP1, GSTA1 et ALT1 ; ADH1 (ADH1B et/ou (ADH1B et ADH1A)), BHMT (variant 1 et/ou un mélange des deux variants 1 et 2), ARG1, FABP1 et GSTA1 ; ADH1 (ADH1B et/ou (ADH1B et ADH1A)), BHMT (variant 1 et/ou un mélange des deux variants 1 et 2), ARG1, FABP1 et ALT1 ; ADH1 (ADH1B et/ou (ADH1B et ADH1A)), BHMT (variant 1 et/ou un mélange des deux variants 1 et 2), ARG1, GSTA1 et ALT1 ; ADH1 (ADH1B et/ou (ADH1B et ADH1A)), BHMT (variant 1 et/ou un mélange des deux variants 1 et 2), FABP1, GSTA1 et ALT1 ou des cinq biomarqueurs ADH1 (ADH1B et/ou (ADH1B et ADH1A)), ARG1, FABP1, GSTA1 et ALT1.

La détection d'une lésion hépatique peut être mise en évidence par la détection et/ou la quantification de six biomarqueurs spécifiquement, tels que ADH1 (ADH1B et/ou (ADH1B et ADH1A)), ADH4, BHMT (variant 1 et/ou un mélange des deux variants 1 et 2), ARG1, FABP1 et GSTA1 ; ADH1 (ADH1B et/ou (ADH1B et ADH1A)), ADH4, BHMT (variant 1 et/ou un mélange des deux variants 1 et 2), ARG1, FABP1 et ALT1 ; ADH1 (ADH1B et/ou (ADH1B et ADH1A)), ADH4, BHMT (variant 1 et/ou un mélange des deux variants 1 et 2), ARG1, GSTA1 et ALT1 ; ADH1 (ADH1B et/ou (ADH1B et ADH1A)), ADH4, BHMT (variant 1 et/ou un mélange des deux variants 1 et 2), FABP1, GSTA1 et ALT1 ; ADH1 (ADH1B et/ou (ADH1B et ADH1A)), ADH4, ARG1, FABP1, GSTA1 et ALT1 ou des six biomarqueurs ADH1 (ADH1B et/ou (ADH1B et ADH1A)), BHMT (variant 1 et/ou un mélange des deux variants 1 et 2), ARG1, FABP1, GSTA1 et ALT1 ;

La détection d'une lésion hépatique peut être mise en évidence par la détection et/ou la quantification de sept biomarqueurs spécifiquement à savoir ADH1 (ADH1B et/ou (ADH1B et ADH1A)), ADH4, BHMT (variant 1 et/ou un mélange des deux variants 1 et 2), ARG1, FABP1, GSTA1 et ALT1.

Dans un mode de réalisation particulier, en plus du marqueur ADH1 (ADH1B et/ou (ADH1B et ADH1A)), le marqueur ADH4 et/ou BHMT sera préférentiellement employé ; en cas de lésion hépatique, la concentration plasmatique/sérique de l'ADH4 et celle de la BHMT est élevée et plus facilement quantifiable.

Dans un mode de réalisation préféré, la détection d'une lésion hépatique est mise en évidence par la détection et/ou la quantification des trois biomarqueurs ADH1 ((ADH1B et/ou (ADH1B et ADH1A)), ADH4 et BHMT (variant 1 et/ou un mélange des deux variants 1 et 2).

Dans un mode préféré, les lésions hépatiques peuvent être détectées par l'analyse de trois biomarqueurs parmi les protéines : ADH1 (ADH1B et/ou ADH1B et ADH1A), ADH4, ARG1, FABP1, GSTA1, ALT1 et BHMT (variant 1 et/ou combinaison des deux variants 1 et 2).

Avantageusement, plus la détection d'une lésion hépatique fait intervenir de biomarqueurs, plus l'analyse du trouble hépatique sera améliorée.

### Procédé permettant la détection spécifique et la quantification absolue de biomarqueurs protéiques

La détection spécifique et la quantification absolue de biomarqueurs protéiques dans les fluides biologiques peut être réalisée de différentes manières. En laboratoire d'analyses médicales, les biomarqueurs sont couramment détectés et quantifiés par des procédés immunologiques. Cela suppose la disponibilité d'un anticorps se liant de manière spécifique à la protéine à doser. La spectrométrie de masse permet de détecter et de doser des molécules, et plus particulièrement des peptides, directement et sans disposer d'un anticorps. Ainsi ont été développées au cours de la dernière décennie des techniques de spectrométrie de masse, notamment des méthodes connues sous les signes SRM (« Selected Reaction Monitoring ») et MRM (« Multiple Reaction Monitoring ») permettant de quantifier une ou plusieurs molécules cibles dans un échantillon biologique complexe.

Cependant, le dosage absolu (c'est-à-dire la détermination de la concentration des protéines cibles) par spectrométrie de masse nécessite des standards de quantification. Il est possible d'utiliser différents types de standards de quantification qui peuvent correspondre à des peptides isotopiquement marqués, des concaténères de peptides marquées, des fragments de protéines isotopiquement marquées ou des protéines entières isotopiquement marquées (voir les publications Brun et al, « Isotope dilution strategies for absolute quantitative proteomics. » Journal of Proteomics (2009) 72(5) pages 740-749 et Zeiler et al, "A Protein Epitope Signature Tag (PrEST) library allows SILAC-based absolute quantification and multiplexed determination of protein copy numbers in cell lines." Molecular and Cellular Proteomics (2012) 11(3):O111.009613). Dans tous les cas, les standards génèrent ou correspondent aux peptides signatures qui sont des peptides issus de la digestion des protéines cibles et qui servent de substituts pour l'analyse de ces protéines en spectrométrie de masse (l'obtention et l'analyse des peptides signatures sont décrites plus loin). Parmi ces approches de quantification, et comme évoqué dans le document WO 2008 / 145 763 A1, l'utilisation conjointe de la spectrométrie de masse en mode SRM ou MRM et de protéines étalons, analogues isotopiquement marqués des biomarqueurs cibles (standards PSAQ™) et introduits en quantité connue dans un échantillon, permet de détecter spécifiquement et de quantifier avec exactitude et précision, dans cet échantillon, les biomarqueurs ciblés.

On entend par « protéine isotopiquement marquée homologue du marqueur visé ou biomarqueur cible » ou « protéine étalon », toute protéine possédant la même séquence en acides aminés que la protéine native que l'on souhaite doser et dont certains atomes sont remplacés par des atomes marqués avec des isotopes stables. Avantageusement, certains acides aminés particuliers peuvent être marqués isotopiquement, tels que l'arginine et la lysine. Dans ce cas, les résidus arginines, et lysines des protéines étalons sont uniformément marquées avec des isotopes du carbone et de l'azote, i.e. du ¹³C et du ¹⁵N. Selon l'invention, avantageusement, le standard est introduit dans l'échantillon sanguin de manière à ce que sa concentration finale dans l'échantillon sanguin soit comprise entre 0,5 et 5 µg/mL, pour les pathologies hépatiques aiguës de type ALI ou ALF. Dans le cas des stéatohépatites non alcooliques ou alcooliques, le standard est introduit à une concentration comprise entre 20 et 200 ng/mL. En fonction de la nature de la protéine ciblée, des concentrations optimales pour la calibration ont été déterminées pour chacune des protéines étalons et présentées ci-dessous dans le tableau 2.

**Tableau 2 : Concentration optimale (µg/mL) de la protéine étalon dans l'échantillon sanguin par type de protéine étalon (cas des atteintes hépatiques aiguës ALI/ALF)**

| Protéine étalon (standard PSAQ™) | Concentration optimale (µg/mL) de la protéine étalon dans l'échantillon sanguin |
|---|---|
| ARG1 | 1 |
| BHMT1 | 2 |
| GSTA1 | 1 |
| ADH1B | 3 |
| ADH4 | 2 |
| FABP1 | 1 |
| ALT1 | 3 |

Dans un mode de réalisation préféré de l'invention, on détecte les biomarqueurs non pas directement mais par l'intermédiaire de sous-unités peptidiques, appelés « peptides signatures ». On entend ici par « peptide signature », tout peptide ayant une séquence d'acides aminés comprenant entre 5 et 30 acides aminés, qui est incluse dans la séquence du marqueur à analyser, permettant d'identifier spécifiquement dans un échantillon sanguin la présence dudit marqueur. Avantageusement, ces peptides signatures sont générés par une digestion enzymatique ou chimique de l'échantillon ou d'une fraction de celui-ci, comme cela sera expliqué plus en détail ci-dessous. Plus particulièrement, les biomarqueurs choisis sont identifiés par la détection de peptides protéolytiques obtenus après digestion des protéines contenues dans l'échantillon sanguin, i.e. peptides signatures permettant la quantification des marqueurs dans l'échantillon sanguin, comme évoqué dans le document WO 2008 / 145 763 A1. Ces peptides signatures permettent de suivre ces biomarqueurs et en particulier leur isoformes prédominantes dans le foie. En effet, certains peptides signatures (cf. figure 8) sont partagés entre plusieurs isoformes, tels que l'ADH1 où le peptide signature INEGFDLLHSGK permet de suivre à la fois l'ADH1B et l'ADH1A, tels que la BHMT avec les isoformes BHMT1 et BHMT2 ou la GSTA avec les isoformes GSTA1, GSTA2, GSTA3 ou GSTA5 (cf. tableau 3, figure 8).

**Tableau 3 : Peptides signatures des biomarqueurs utilisés dans le procédé selon l'invention et générés par une digestion avec de la trypsine ou un mélange de trypsine et endolysC**

| Biomarqueur d'intérêt | Peptide signature du marqueur d'intérêt | Identification des séquences peptidiques (SEQ ID N°) | Spécificité du peptide par rapport au biomarqueur |
|---|---|---|---|
| ADH1B | AAVLWEVK | SEQ ID N° 17 | spécifique |
| ADH1B | INEGFDLLHSGK | SEQ ID N° 16 | peptide présent dans ADH1A et ADH1B |
| ADH1B | FSLDALITHVLPFEK | SEQ ID N° 15 | peptide présent dans ADH1A et ADH1B |
| ADH1B | IDAASPLEK | SEQ ID N° 14 | peptide présent dans ADH1A, ADH1B, ADH1C et ADH1G |
| ADH4 | FNLDALVTHTLPFDK | SEQ ID N° 20 | spécifique |
| ADH4 | IDDDANLER | SEQ ID N° 18 | spécifique |
| ADH4 | IIGIDINSEK | SEQ ID N° 19 | spécifique |
| ARG1 | DVDPGEHYILK | SEQ ID N° 25 | spécifique |
| ARG1 | TIGIIGAPFSK | SEQ ID N° 24 | spécifique |
| ARG1 | EGLYITEEIYK | SEQ ID N° 26 | spécifique |
| BHMT1 | EATTEQQLK | SEQ ID N° 21 | spécifique |
| BHMT1 | AIAEELAPER | SEQ ID N° 23 | peptide présent dans BHMT1 et BHMT2 |
| BHMT1 | EAYNLGVR | SEQ ID N° 22 | peptide présent dans BHMT1 et BHMT2 |
| FABP1 | AIGLPEELIQK | SEQ ID N° 27 | spécifique |
| FABP1 | FTITAGSK | SEQ ID N° 28 | spécifique |
| FABP1 | TVVQLEGDNK | SEQ ID N° 29 | spécifique |
| GSTA1 | LHYFNAR | SEQ ID N° 30 | spécifique |
| GSTA1 | SHGQDYLVGNK | SEQ ID N° 31 | peptide présent dans GSTA1, GSTA2 et GSTA3 |
| GSTA1 | ISNLPTVK | SEQ ID N° 33 | peptide présent dans GSTA1, GSTA2, GSTA3 et GSTA5 |
| GSTA1 | AILNYIASK | SEQ ID N° 32 | peptide présent dans GSTA1, GSTA2, GSTA3 et GSTA5 |
| ALT1 | ALELEQELR | SEQ ID N° 34 | spécifique |
| ALT1 | LLVAGEGHTR | SEQ ID N° 35 | spécifique |
| ALT1 | KPFTEVIR | SEQ ID N° 36 | peptide présent dans ALT1 et ALT2 |

Après introduction dans un échantillon sanguin d'une quantité connue de protéine étalon PSAQ™, identique ou structurellement très proche de la protéine native que l'on souhaite doser, une étape de digestion, enzymatique ou chimique, peut être réalisée de manière à obtenir les peptides protéolytiques des protéines initialement présentes dans l'échantillon dont les peptides signatures des biomarqueurs cibles facilement identifiables et quantifiables par LC-MS en mode SRM ou MRM. Il est également possible d'utiliser, à la place des standards PSAQ, des standards peptidiques isotopiquement marqués (appelés peptides AQUA pour "Absolute quantification" ou SIL pour "Stable Isotope Labelling". Ces peptides étalons marqués ont une séquence identique aux peptides signatures suivis et ils sont ajoutés dans l'échantillon juste avant ou juste après l'étape de digestion des protéines. Ces standards permettent une quantification absolue des protéines cibles mais qui est souvent moins exacte que la quantification par la méthode PSAQ.

Avantageusement, la digestion des protéines est effectuée de manière enzymatique, et plus particulièrement en présence d'une solution de trypsine ou bien un mélange de trypsine et d'EndolysC. La quantité d'enzyme utilisée pour la digestion correspond généralement à un ratio (en poids) enzyme/protéines de 1/100 à 1/10. Après addition de la solution d'enzyme dans l'échantillon sanguin et incubation à 37°C, de manière à générer les peptides protéolytiques, la digestion est stoppée par addition d'au moins une quantité suffisante d'acide formique dans l'échantillon sanguin. On entend par « peptide protéolytique » tout peptide ou séquence d'acides aminés issu(e) de la dégradation d'un polypeptide, notamment par digestion enzymatique ou chimique dudit polypeptide.

Les peptides protéolytiques (dont les peptides signatures et les peptides isotopiquement marqués) ainsi obtenus sont ensuite analysés par LC-MS. A noter qu'il est possible d'utiliser différents types d'enzymes pour effectuer la digestion des protéines. En fonction de la spécificité de clivage de l'enzyme choisie, les peptides signatures permettant de détecter et quantifier les protéines cibles seront différents. Ces peptides signatures peuvent être prédits en effectuant une digestion *in silico* des protéines cibles (dont on connait la séquence en acides aminés). Leur détectabilité en spectrométrie de masse est ensuite vérifiée expérimentalement. Le tableau 3 répertorie des peptides signatures obtenus et suivis après une digestion avec de la trypsine ou un mélange de trypsine et d'endolysC. L'endolysC est une endoprotéinase Lys-C.

Afin de faciliter l'isolation et l'identification des protéines présentes dans les échantillons sanguins, une étape de prétraitement de l'échantillon sanguin peut avantageusement être réalisée. En effet, cette étape permet d'éliminer au moins en partie des protéines abondantes non ciblées présentes dans l'échantillon sanguin et ainsi d'accroitre la sensibilité de détection des biomarqueurs cibles tout en conservant l'intégrité du standard PSAQ™ (protéine étalon) et du biomarqueur cible.

De manière à conserver, au cours du procédé, l'intégrité des biomarqueurs cibles et des standards PSAQ™, plusieurs modes de prétraitement peuvent être utilisés, tels que la déplétion des protéines abondantes par l'utilisation d'un dispositif de chromatographie d'affinité (immunodéplétion), un réactif chimique ayant une affinité pour l'albumine (type cibacron blue) ou un procédé de glycodéplétion de l'échantillon sanguin (cf. figure 1). Ces méthodes de prétraitement sont connues en tant que telles.

La déplétion des protéines abondantes est une méthode d'affinité permettant d'extraire au moins en partie les protéines majoritaires du sérum, i.e. les protéines abondantes telles que l'albumine, l'IgG, la transferrine ou encore l'IgA.

La glycodéplétion est une méthode permettant de capturer et d'extraire au moins en partie les glycoprotéines présentes dans l'échantillon sanguin de manière à laisser dans l'échantillon sanguin les autres protéines telles que les biomarqueurs cibles qui ne sont pas glycosilés. Dans le procédé qui fait l'objet du schéma selon la figure 1, la capture des glycoprotéines est réalisée par des billes magnétiques fonctionnalisés par de l'hydrazide.

Afin d'éviter d'effectuer une détection sensible et une quantification exacte des biomarqueurs cibles on choisit un mode de prétraitement de l'échantillon adapté à chaque biomarqueur. Ainsi, les protéines ARG1, ADH1 (variant A et B), BHMT (variants 1 et 2) et GSTA1 seront préférentiellement traités par déplétion des protéines abondantes par chromatographie d'affinité et les protéines ADH4, FABP1, ALT1 et BHMT par glycodéplétion.

Après traitement de l'échantillon sanguin, une étape additionnelle de traitement de l'échantillon sanguin peut être effectuée par électrophorèse. Cette étape permet d'affiner l'extraction des protéines d'intérêt. Cette étape d'électrophorèse peut être effectuée sur gel d'acrylamide. Dans ce cas, la digestion enzymatique est également réalisée en gel.

Une étape additionnelle d'enrichissement des peptides cibles par immunoaffinité avant toute analyse par LC-MS en mode SRM ou MRM peut avantageusement être réalisée. Cela permet de faciliter la détection et la quantification de ces peptides par spectrométrie de masse.

Les peptides protéolytiques sont ensuite analysés par LC-MS en mode SRM. Des courbes d'étalonnage ont été réalisées pour chaque biomarqueur de manière à évaluer les performances analytiques du dosage et garantir la fiabilité de la quantification des biomarqueurs cibles dans l'échantillon sanguin. Les courbes d'étalonnages ont été réalisées en employant des échantillons contenant une quantité croissante de protéine non marquée et une quantité constante de protéine standard PSAQ™, ces échantillons ayant subis les mêmes traitements que l'échantillon sanguin dont on souhaite détecter et quantifier un biomarqueur cible, à savoir des prétraitements et/ou une digestion. Des blancs contenant uniquement les protéines endogènes sont aussi réalisés. Tous les points de calibration sont effectués plusieurs fois par souci de reproductibilité (cf. figures 2 et 3).

Les résultats de quantification obtenus pour les différents peptides signatures de chaque biomarqueur cible sont cohérents et montrent que le procédé selon l'invention permet de détecter et de quantifier avec précision des biomarqueurs cibles. Les résultats de quantification sont présentés ci-après dans le tableau 4 pour chaque biomarqueur et pour chaque peptide signature obtenu après immunodéplétion ou glycodéplétion. Le tableau 4 présente la limite inférieure de détection (LID) des différentes protéines déterminée par les courbes d'étalonnage précédemment décrites et la limite inférieure de quantification (LIQ) définie comme étant égale à 3 fois la LID.

Les analyses LC-SRM ont été réalisées sur un spectromètre de masse de type QTRAP dans une gamme allant de 400 à 1000 m/z. Avant toute analyse, les peptides protéolytiques peuvent être concentrés sur une précolonne et séparées sur colonne de chromatographie liquide en mode gradient de manière à faciliter l'analyse ultérieure

L'identification des pics de chaque peptide a été réalisée en utilisant le logiciel Skyline, connus des spécialistes de la spectrométrie de masse. En plus de l'évaluation du signal du peptide (signal composite), toutes les transitions sont inspectées individuellement et exclues si elles sont jugés inaptes pour la quantification (faible rapport signal sur bruit, interférences évidentes). Les ratios des surfaces des pics relatifs aux peptides marqués et non marqués sont calculés pour chaque transition SRM. Ces ratios sont ensuite utilisés pour déterminer le ratio moyen correspondant, et finalement le rapport moyen de l'abondance de la protéine cible est calculé à partir des rapports obtenus pour les différents peptides signatures. La concentration des biomarqueurs a ensuite été calculée à partir du ratio moyen obtenu pour chacune des protéines. La limite inférieure de détection (LID) a été déterminée selon la méthode de la courbe d'étalonnage et la limite inférieure de quantification (LIQ) est définie comme étant égale à 3 fois la LID. L'. Pour chaque patient, la concentration des biomarqueurs cibles est déterminée à différents instants t pendant leur hospitalisation.

**Tableau 4 : Performance analytique du test pour chaque biomarqueur ciblé (limite de détection).**

| Protéine | Peptide signature | Méthode biochimique | Gamme de concentrations testées [µg/ml] | Précision [%] | LID [µg/ml] | LIQ [µg/ml] | Précision à LIQ (CV en %) |
|---|---|---|---|---|---|---|---|
| ADH1B | INEGFDLLHSGK | MARS | 0,81-73,5 | 79 | 10.66 | 31.99 | 41 |
| | IDAASPLE**K** | | | 95 | 0.72 | 2.17 | 22-26 |
| | | | | 97 | 0.77 | 2.30 | ND |
| | AAVLWEVK | | | 112 | 2.01 | 6.03 | 3-13 |
| ARG1 | DVDPGEHYILK | MARS | 0,04-1,50 | 146 | 0.31 | 0.94 | 9 |
| | TIGIIGAPFSK | | | 118 | 0.30 | 0.90 | 4-8 |
| | EGLYITEEIYK | | | 138 | 0.31 | 0.92 | 6-15 |
| BHMT1 | AIAEELAPER | MARS | 1,50-145,8 | 114 | 1.38 | 4.15 | 12-19 |
| | EAYNLGVR | | | 88 | 3.49 | 10.48 | 15-40 |
| | EATTEQQLK | | | 83 | 2.04 | 6.11 | 6-15 |
| GSTA1 | ISNLPTVK | MARS | 0,09-3,43 | 121 | 0.30 | 0.91 | 6-10 |
| | LHYFNAR | | | 132 | 0.32 | 0.97 | 10-14 |
| | AILNYIASK | | | 124 | 0.29 | 0.87 | 7-10 |
| | SHGQDYLVGNK | | | 125 | 0.41 | 1.24 | 9-11 |
| ADH4 | | Glycodéplétion | 0,25-24,8 | 139 | 1.59 | 4.77 | 9-16 |
| | IDDDANLER | | | 148 | 1.75 | 5.25 | 8-10 |
| | IIGIDINSEK | | | 136 | 1.60 | 4.81 | 0-8 |
| BHMT1 | AIAEELAPER | Glycodéplétion | 0,60-79,5 | 143 | 6.53 | 19.58 | 8 |
| | EAYNLGVR | | | 151 | 4.15 | 12.45 | NA |
| | EATTEQQLK | | | 136 | 6.17 | 18.52 | 2-6 |
| FABP1 | AIGLPEELIQK | Glycodéplétion | 0,18-3,69 | 110 | 0.71 | 2.13 | 2-13 |
| | FTITAGSK | | | 114 | 0.64 | 1.91 | 5-14 |
| | TVVQLEGDNK | | | 108 | 0.66 | 1.99 | 1-8 |
| ALT1 | ALELEQELR | Glycodéplétion | 0,40-8,10 | 96 | 1.21 | 3.62 | 6-14 |
| | LLVAGEGHTR | | | 92 | 1.10 | 3.29 | 15 |
| | KPFTEVIR | | | 117 | 2.17 | 6.52 | 9-10 |

Chez les patients atteints d'hépatites aiguës (ALI) ou fulminantes (ALF), les concentrations sériques des biomarqueurs (en particulier ADH1B, ADH1A et ADH1B, ADH4 et BHMT1, BHMT1 et BHMT2) s'élèvent rapidement pendant la phase aigüe et chutent à des niveaux très bas pendant la régénération hépatique (figures 4 et 5). Cette cinétique sérique est corrélée à l'évolution des paramètres classiques de suivi biologique (paramètres de coagulation, transaminases et CK18) mais les concentrations des biomarqueurs ADH1B, ADH1B et ADH1A, ADH4, BHMT1, BHMT1 et BHMT2 se rétablissent plus précocement. Ces résultats suggèrent que ces biomarqueurs peuvent être très utiles pour améliorer le suivi biologique du patient, évaluer plus précocement le pronostic (régénération hépatique ou aggravation) et aider à la décision de transplantation hépatique. Dans le cas des hépatites aiguës ou fulminantes induites par le paracétamol, les rapides et fortes augmentations des concentrations sériques des biomarqueurs peuvent être utiles à l'identification précoce de l'étiologie, ce qui est déterminant pour l'administration rapide du traitement et un pronostic favorable.

Ces biomarqueurs peuvent être également utilisés pour l'évaluation de la toxicité de molécules chimiques (candidats médicaments), de médicaments (pharmacovigilance) ou autres xénobiotiques sur l'homme ou sur des modèles animaux, ou sur des modèles *in vitro* (cultures de lignées cellulaires hépatiques). Chez l'homme et les animaux, le dosage peut être effectué sur des échantillon sanguins. Dans le cas des modèles *in vitro,* ce dosage sera effectué à partir du milieu de culture.

### Exemples

L'invention est illustrée ci-dessous par des exemples qui cependant ne limitent pas l'invention. Ces exemples portent sur une méthode de diagnostic *in vitro* d'atteintes hépatiques à partir d'un échantillon sanguin et son utilisation, notamment pour déceler une insuffisance hépatique aiguë ou une stéatohépatite non alcoolique chez un patient.

### 1. Préparation de la protéine étalon

Des protéines recombinantes non marquées ont été achetées chez Abcam. Des protéines marquées isotopiquement (standards PSAQ™) ont été synthétisées en utilisant : soit un système d'expression de protéines acellulaire en présence de [¹³C₆, 1⁵N₂] L-lysine et [¹³C₆, ¹⁵N₄] L-arginine (Eurisotop, Saint Aubin, France), comme décrit dans la publication de Lebert et al., « Production and use of stable isotope-labeled proteins for absolute quantitative proteomics » parue dans Methods in Molecular Biology vol.753, p.93-115 (2011), soit un système d'expression correspondant à une souche d'Escherichia coli auxotrophe pour la lysine et l'arginine comme décrit dans l'article de Picard et al., « PSAQ™ standards for accurate MS-based quantification ofproteins: from the concept to biomedical applications » J Mass Spectrom. 2012;47(10), p.1353-1363.

La pureté des protéines étalon a été vérifiée (> 95% de pureté) et quantifiée par analyse d'acides aminés comme décrit dans la publication de Louwagie et al. « Introducing AAA-MS, a rapid and sensitive method for amino acid analysis using isotope dilution and high-resolution mass spectrometry » parue dans Journal of Proteome Research vol.11, p3929-3936 (2012). Pour ce faire, les protéines PSAQ™ ont été hydrolysées en voie acide en présence d'une quantité connue d'une protéine de référence l'albumine de sérum bovin fournie par l'Institut National des Standards et de la Technologie (Etats-Unis). Les acides aminés issus des protéines PSAQ™ ont été quantifiés par spectrométrie de masse haute résolution par comparaison des signaux émis par les acides aminés marqués issus de la protéine PSAQ™ et leurs analogues non marqués issus de l'albumine de sérum bovin. L'incorporation des acides aminés isotopiquement marqués, tel que déterminée par LC-MS et LC-SRM (chromatographie en phase liquide couplée à la spectrométrie de masse en mode de suivi de réaction sélectionnée), était supérieure à 99%.

### 2. Préparation d'un échantillon biologique

Treize patients porteurs d'une insuffisance hépatique aiguë (ALI) ont été recrutés au département d'hépatologie de l'Hôpital Paul Brousse (Villejuif, France). L'insuffisance hépatique aiguë de ces patients a été décelée par une activité élevée de transaminase reflétant la lyse active des cellules du foie présente dans le sang de ces patients, des troubles de la coagulation avec un INR (International Normalized Ratio) > 1.5 et par l'absence chez ces patients de maladie hépatique préexistante.

Parmi ces patients, quatre ont présenté une encéphalopathie hépatique (grade >=1) en moins de 26 semaines après l'apparition des symptômes et ont été classés comme patients ALF (cf. tableau 5). Des échantillons de sérum de ces patients ont été fournis par le Centre de Ressources Biologiques de la Faculté Paris-Sud de Médecine, France (numéro d'agrément: 2011/39938). L'Etablissement Français du Sang (Etablissement Français du Sang, La Tronche, France) a fourni sept échantillons de sérum anonymes provenant de donneurs sains. Ces échantillons de sérum ont ensuite été recueillis dans des tubes non traités (BD Biosciences, Le Pont de Claix, France) et ont été centrifugés à 1000 g pendant 15 min pour obtenir le sérum. Des échantillons de sérum ont été aliquotés et immédiatement congelés à - 80 °C avant toute utilisation ultérieure. Pour chaque patient, plusieurs échantillons de sérum ont été prélevés à plusieurs jours d'échantillonnage pendant l'hospitalisation. Des analyses ont été faites sur chaque patient de manière à s'assurer que ces patients ne souffraient pas d'une hépatite virale A, B et/ou C. Tous les patients ont été criblés pour l'hépatite virale A, B et C (HAV, HBV, HCV) et ont été trouvés négatifs pour tous ces virus. Les paramètres biologiques de l'atteinte hépatique (INR, le temps de prothrombine (PT), les niveaux de transaminases, de bilirubine, de plaquettes et de créatinine) ont été déterminés en utilisant des méthodes standard. Les niveaux de la protéine CK18 soluble et de la protéine caspase - cytokératine-18 clivée (CK18Asp396) présentes dans le sérum ont été mesurés à l'aide des kits ELISA M65 et M30Apoptosense (Peviva, Suède). Les courbes d'étalonnage ont été établies en utilisant le plasma humain acheté auprès de Sigma Aldrich (Saint Quentin Fallavier, France).

**Tableau 5 : Caractéristiques cliniques et biologiques des patients atteints d'ALI (Insuffisance hépatique aiguë) ou d'ALF**

| **Numéro du patient** | **Collecte des échantillons à x j après l'hospitalisation** | **Aetiologie** | **Sexe** | **Age (années)** | **activité ALT (U/L)** | **PI* (%)** | **INR**** | **ALI or ALF***** | **Résultat** | **CK18 (U/L)** | **CK18 Asp396 (U/L)** |
|---|---|---|---|---|---|---|---|---|---|---|---|
| 1 | 0 | Paracétamol | F | 20 | 1489 | 9 | 16 | ALI | Vivant | 13800 | 3333 |
| | 10 | | | | 402 | 59 | 1.42 | | | 1048 | 944 |
| 2 | 0 | Paracétamol | F | 29 | 634 | 19 | 4.8 | ALF | Vivant | N/A | N/A |
| | 2 | | | | 4565 | 17 | 5.45 | | | N/A | N/A |
| | 3 | | | | 8118 | 18 | 4.73 | | | N/A | N/A |
| 3 | 0 | Paracétamol | F | 24 | 6410 | 21 | 3.82 | ALI | Vivant | 22243 | 5691 |
| | 3 | | | | 2295 | 66 | 1.31 | | | 1150 | 404 |
| 4 | 0 | Paracétamol | F | 18 | 2653 | 31 | 2.6 | ALI | Vivant | 19806 | 7212 |
| | 2 | | | | 1900 | 57 | 1.45 | | | 1618 | 479 |
| 5 | 0 | Paracétamol | M | 25 | 173 | 41 | 1.99 | ALI | Vivant | N/A | N/A |
| | 2 | | | | 2571 | 26 | 3.21 | | | N/A | N/A |
| | 6 | | | | 683 | 75 | 1.2 | | | N/A | N/A |
| 6 | 0 | Paracétamol | F | 23 | 770 | 39 | 2.06 | ALI | Vivant | 5138 | 1379 |
| | 1 | | | | 7165 | 18 | 4.8 | | | 23088 | 8938 |
| | 5 | | | | 1831 | 89 | 1.07 | | | 956 | 529 |
| 7 | 0 | Médicament + alcool | M | 24 | 7300 | 13 | 7.47 | ALI | Vivant | 12138 | 5350 |
| | 7 | | | | 481 | 100 | 0.89 | | | 631 | 338 |
| 8 | 0 | Paracétamol | F | 16 | 15480 | 20 | 4.17 | ALI | Vivant | 21363 | 533 |
| | 4 | | | | NA | 68 | 1.3 | | | 600 | 254 |
| 9 | 0 | Paracétamol + alcool | M | 57 | 2710 | 16 | 5.53 | ALF | Mort (sepsis) | 23025 | 15288 |
| | 2 | | | | NA | 42 | 1.97 | | | 9956 | 1117 |
| | 7 | | | | NA | 51 | 1.65 | | | 2000 | 421 |
| 10 | 0 | maladie auto-immune | F | 58 | 2224 | 17 | 5.76 | ALF | Vivant | 15638 | 9933 |
| | 2 | | | | 1861 | 11 | 10.69 | | | 15013 | 7275 |
| | 5 | | | | NA | 66 | 1.3 | | | 1275 | 450 |
| 11 | 0 | maladie auto-immune | M | 56 | 2170 | 31 | 2.52 | ALI | Vivant | 23056 | 15117 |
| | 2 | | | | 1119 | 16 | 5.59 | | | 6400 | 2417 |
| | 12 | | | | NA | 85 | 1.11 | | | 2256 | 617 |
| 12 | 0 | Virus Herpès simplex | F | 22 | 1383 | 14 | 6.52 | ALF | Vivant | 11669 | 4863 |
| | 5 | | | | 153 | 40 | 2.07 | | | 11388 | 4029 |
| | 15 | | | | 133 | 56 | 1.1 | | | 1088 | 229 |
| 13 | 0 | Médicament | F | 32 | 892 | 28 | 2.8 | ALI | Vivant | 14781 | 7458 |
| | 2 | | | | 984 | 54 | 1.58 | | | 3463 | 938 |
| | 3 | | | | 589 | 80 | 1.15 | | | 1856 | 375 |

### 3. Procédé de diagnostic in vitro de troubles hépatiques par la présence et la quantification d'un marqueur d'intérêt

La protéine-étalon PSAQ™ est injectée directement dans l'échantillon de sérum avant tout prétraitement. Cette approche permet de déterminer, dans des fluides biologiques sanguins tels que le plasma et le sérum, les concentrations de certaines protéines cibles tels que des biomarqueurs de pathologies dont ils constituent des analogues marqués. Plusieurs modes de prétraitement tels que la déplétion des protéines abondantes (par immunoaffinité ou affinité avec un réactif chimique) et la glycodéplétion de l'échantillon sanguin ont été envisagés de manière à conserver, au cours du procédé, l'intégrité des protéines PSAQ™. Ainsi, les protéines ARG1, ADH1B, BHMT1 et GSTA ont été sujettes à la déplétion des protéines abondantes selon la technologie MARS d'Agilent Technologies et les protéines ADH4, FABP1, ALT1 et BHMT1 à la glycodéplétion.

### a. Déplétion des protéines abondantes

Avant tout prétraitement, des quantités définies de protéine PSAQ™ (voir tableau 2) ont été ajoutées à 14 µl de sérum ou de plasma précédemment prélevés sur un individu, qu'il soit porteur d'une insuffisance hépatique aiguë ou donneur sain de manière à pouvoir quantifier la présence des protéine dans le sérum de l'individu. La solution sérum - protéines PSAQ™ a ensuite été laissée incubée pendant 1 heure à 4 °C sous agitation douce.

L'échantillon comprenant du sérum (ou du plasma) et les protéines PSAQ™, ont été déposés sur une colonne d'affinité permettant d'extraire les six protéines les plus abondantes (Albumine, IgG, Transferrine, IgA, haptoglobine et antitrypsine) dans le sérum (technologie MARS, cartouche spin d'Agilent Technologies, Les Ulis, France) selon les instructions du fabricant. Cette méthode permet d'extraire près de 85 à 90% de l'ensemble des protéines présentes dans le sérum.

Suite au passage du sérum (ou plasma) sur la colonne d'affinité MARS, les échantillons de sérum ainsi exempts d'albumine, d'IgG, de transferrine, d'IgA, d'haptoglobine et de antitrypsine se sont retrouvés concentrés jusqu'à un volume de 50 µl sur un dispositif d'ultrafiltration possédant une limite d'exclusion de 3000 Da (Merck Millipore, Molsheim, France). Une solution d'urée 4 M et de NH₄HCO₃ à 50 mM a été déposée sur l'échantillon dans le dispositif d'ultrafiltration de manière à échanger le tampon.

Les concentrés de protéines faiblement abondantes résultants ont ensuite été soumis à une étape de digestion enzymatique

### b. Glycodéplétion

Avant tout prétraitement, des quantités définies de protéine PSAQ™ (voir tableau 2) ont été ajoutées à 18 µl de sérum ou de plasma précédemment prélevés sur un individu, qu'il soit porteur d'une insuffisance hépatique aiguë ou donneur sain de manière à pouvoir quantifier la présence de cette protéine dans le sérum du patient. La solution sérum - protéine PSAQ™ a ensuite été laissée incuber pendant 1 heure à 4 °C sous agitation douce.

L'échantillon de sérum ou de plasma précédemment prélevé sur un patient, qu'il soit porteur d'une insuffisance hépatique aiguë ou donneur sain, et additionné de standards PSAQ, a été mélangé avec 11 µl d'une solution de meta-periodate de sodium à 0.1 M afin d'oxyder les glycoprotéines et de transformer les groupements cis-diol des sucres en aldéhydes. L'échantillon comportant des glycoprotéines porteuses de groupements aldéhydes et des protéines non glycosilées a été introduit sur une colonne d'affinité du kit d'immobilisation GlycoLink de Life technologies selon les instructions du fabricant. La colonne d'affinité employée comprend une résine UltraLink fonctionnalisée par de l'hydrazide. La résine ainsi fonctionnalisée va permettre d'extraire les glycoprotéines présentes dans le sérum par la réalisation de liaisons stables entre l'hydrazide de la résine et les aldéhydes des glycoprotéines précédemment modifiées. Les protéines non glycosylées ont ensuite été éluées par centrifugation en présence de 400 µl d'urée à une concentration de 8M. L'échantillon a été concentré jusqu'à 50 µL sur un dispositif d'ultrafiltration possédant une limite d'exclusion de 3000 Da puis a été soumis à la digestion.

### Courbes d'étalonnage

Afin de pouvoir quantifier la présence d'une protéine cible dans un échantillon de sérum humain, des courbes d'étalonnage ont été réalisées après les étapes de prétraitement de l'échantillon sanguin (Figures 2 et 3). Pour chaque courbe, 5 points de calibration ont été réalisés par ajout d'une quantité croissante de protéine non marquée et d'une quantité constante de protéine étalon PSAQ™ aux échantillons de plasma. La quantité de protéines non marquées ajoutée a été ajustée de manière à couvrir tous les concentrations sériques/plasmatiques les plus extrêmes dans la pathologie étudiée. Des blancs contenant uniquement les biomarqueurs endogènes ont aussi été réalisés. Tous les points de calibration ont été effectués au moins 3 fois par souci de reproductibilité. Pour les 2 plus faibles points de calibrations, 4 réplicas ont été réalisés. Ces courbes d'étalonnage ont permis d'évaluer les performances analytiques du dosage (linéarité de la réponse, précision, exactitude, sensibilité). Comme le montrent les Figures 2 et 3, le dosage des protéines cibles est très performant et il peut être appliqué à l'analyse d'échantillons cliniques.

### Protéolyse

Une solution de digestion a été préparée en utilisant un mélange d'EndolysC et de trypsine (Promega, Charbonnières-les-Bains, France) à un rapport protéine / enzyme de 1 / 30 en masse dans une solution d'urée 4 M et de NH₄HCO₃ à 50 mM à 37 °C.

Après 3 heures d'incubation, les échantillons ont été dilués quatre fois et incubées une nuit à 37 °C. La digestion a été stoppée par addition d'acide formique (0,1% en concentration finale). Les échantillons ont été purifiés sur colonne SpinColumns macro C18 (Harvard Apparatus, Les Ulis, France) et séchés par centrifugation sous vide. Les culots ont été resolubilisés dans 15 µL d'une solution contenant 2% en volume d'acétonitrile, d'acide formique à 0,1% en volume.

6 µl de cette solution ont été injectés sur le système de chromatographie liquide couplé au spectromètre de masse en mode SRM (LC-SRM).

### Analyse LC-SRM

Les analyses LC-SRM ont été réalisées sur un spectromètre de masse de type 6500 QTrap (AB Sciex, Les Ulis, France) équipé d'une source TurboV et contrôlé par le logiciel Analyst (version 1.6.1, AB Sciex) dans une gamme allant de 400 à 1000 m/z. L'instrument a été couplé à un système de chromatographie Ultima 3000 LC (Thermo Scientific, Courtaboeuf, France). La chromatographie des échantillons a été effectuée en utilisant un système combinant 2 solvants, le solvant A (2% d'acétonitrile et acide formique à 0,1%) et le solvant B (80% acétonitrile, acide formique% 0,1%). Les peptides protéolytiques ont été concentrées sur une précolonne 1 x 15 mm C18 PepMap (Thermo Scientific) puis ont été séparées sur une colonne Kinetex XB-C18 (2,1 x 100 mm, 1,7 µm, 100 Å; Phenomenex, Le Pecq, France) en mode gradient. Ainsi l'élution graduée a été réalisée de manière à modifier la concentration de la solution initiale de B à 3% à 35% en 30 minutes, puis de 35% à 90% en 10 minutes, et ce à un débit de 50 µl/min.

L'analyse des données LC-SRM et l"identification des pics de chaque peptide a ensuite été réalisée en utilisant le logiciel Skyline. En plus de l'évaluation du signal du peptide (signal composite issu de plusieurs transitions), toutes les transitions ont été inspectées individuellement et exclues si elles étaient jugés inaptes pour la quantification (faible rapport signal sur bruit, interférences évidentes). Les ratios des surfaces des pics relatifs aux peptides marqués et non marqués ont été calculés pour chaque transition SRM. Ces ratios ont ensuite été utilisés pour déterminer le ratio moyen correspondant, et finalement le rapport moyen de l'abondance de la protéine cible a été calculé à partir des rapports obtenus pour les différents peptides signatures. La concentration des biomarqueurs a ensuite été calculée à partir du ratio moyen en protéines. La limite inférieure de détection (LID) a été déterminée selon la méthode de la courbe d'étalonnage et la limite inférieure de quantification (LIQ) a été définie comme étant égale à 3 fois la LID. Pour chaque patient, la concentration des biomarqueurs cibles a été déterminée à différents instants t. Les Figures 4 et 5 montent le suivi longitudinal des concentrations sériques des différents biomarqueurs chez des patients atteint d'ALI ou ALF et dont l'étiologie est soit le paracétamol (figure 4) soit une autre cause (figure 5). (cf. figures 4 et 5). Ce suivi est analysé en parallèle avec les paramètres clinico-biologiques standards (taux de transaminases, paramètres de coagulation). Chez les patients avec une ALI/ALF induite par le paracétamol (figure 4), les concentrations sériques des biomarqueurs augmentent avec le développement de l'atteinte hépatique, en particulier les biomarqueurs ADH1B, ADH1B et ADH1A, BHMT1, BHMT1 et BHMT2, et ADH4 qui atteignent des concentrations circulantes très élevées. Les concentrations baissent alors que l'état clinique et les paramètres clinico-biologiques classiques s'améliorent. Pour les patients atteints d'ALI/ALF d'étiologies variées, ces modifications sont également observables mais les niveaux sériques détectés sont globalement moins élevés. La figure 7 concerne des patients atteints de NASH. Chez ces patients le dosage des biomarqueurs cibles en phase aigüe de la maladie montre des concentrations sériques de BHMT1, ADH1A et ADH1B significativement plus élevées que chez des donneurs sains. Cette observation montre que les biomarqueurs cibles présentent un intérêt dans le diagnostic des NASH.

## Revendications

1. Procédé de diagnostic, de suivi, de pronostic et/ou théranostique *in vitro* de troubles hépatiques choisis parmi l'insuffisance hépatique aigüe, la stéatose hépatique et la stéatohépatite, à partir d'un échantillon sanguin issu d'un individu, dans lequel on détermine la présence et/ou la concentration du marqueur ADH1B (SEQ ID N° 2) et/ou la présence et/ou la concentration de la combinaison des marqueurs ADH1B (SEQ ID N° 2) et ADH1A (SEQ ID N° 1).

2. Procédé *in vitro* selon la revendication 1, dans lequel on détermine la présence et/ou la concentration d'au moins un marqueur supplémentaire, préférentiellement d'au moins deux marqueurs supplémentaires, et encore plus préférentiellement d'au moins trois marqueurs supplémentaires, lesdits marqueurs étant sélectionnés dans le groupe constitué par : ADH4 (SEQ ID N° 3 & SEQ ID N° 4), BHMT (SEQ ID N° 5, SEQ ID N° 6 & SEQ ID N° 7), ARG1 (SEQ ID N° 8, SEQ ID N° 9 & SEQ ID N° 10), FABP1 (SEQ ID N° 11), GSTA1 (SEQ ID N° 12) et ALT1 (SEQ ID N° 13).

3. Procédé *in vitro* selon l'une quelconque des revendications 1 à 2, dans lequel on détermine au moins la présence et/ou la concentration d'au moins un marqueur supplémentaire sélectionné parmi ADH4 (SEQ ID N° 3 & SEQ ID N° 4) et BHMT (SEQ ID N° 5, SEQ ID N° 6 & SEQ ID N° 7).

4. Procédé *in vitro* selon l'une quelconque des revendications 1 à 3, dans lequel on détermine au moins la présence et/ou la concentration du marqueur ADH1B (SEQ ID N° 2) ou de la combinaison des marqueurs ADH1B (SEQ ID N° 2) et ADH1A (SEQ ID N° 1), d'une part, et des marqueurs ADH4 (SEQ ID N° 3 & SEQ ID N° 4) et BHMT (SEQ ID N° 5, SEQ ID N° 6 & SEQ ID N° 7), d'autre part.

5. Procédé *in vitro* selon l'une quelconque des revendications 2 à 4 **caractérisé en ce que** le marqueur BHMT est le variant de séquence BHMT1 (SEQ ID N° 5) et/ou la combinaison des variants de séquence BHMT1 (SEQ ID N° 5) et BHMT2 (SEQ ID N° 6 & SEQ ID N° 7).

6. Procédé *in vitro* selon l'une quelconque des revendications 1 à 5, dans lequel on détermine en outre la présence et/ou la concentration du marqueur CK18.

7. Procédé *in vitro* selon l'une quelconque des revendications 1 à 6, dans lequel on utilise pour ladite détermination de la présence et/ou de la concentration desdits marqueurs des peptides signatures présents dans lesdits marqueurs.

8. Procédé *in vitro* selon la revendication 7, dans lequel lesdits peptides signatures sont générés à partir desdits marqueurs par un procédé de digestion, de préférence enzymatique.

9. Procédé *in vitro* selon la revendication 7 ou 8, dans lequel la présence et/ou la concentration:
a. de la combinaison des marqueurs ADH1A (SEQ ID N° 1) et ADH1B (SEQ ID N° 2), si elle est sélectionnée, est déterminée par la présence d'au moins un peptide signature choisi parmi INEGFDLLHSGK (SEQ ID N° 16) et FSLDALITHVLPFEK (SEQ ID N° 15),
b. du marqueur ADH1B (SEQ ID N° 2), s'il est sélectionné, est déterminée par la présence du peptide signature peptide signature AAVLWEVK (SEQ ID N° 17),
c. du marqueur ADH4 (SEQ ID N° 3 & SEQ ID N° 4), s'il est sélectionné, est déterminée par la présence d'au moins un peptide signature choisi parmi FNLDALVTHTLPFDK (SEQ ID N° 20), IDDDANLER (SEQ ID N° 18) ou IIGIDINSEK (SEQ ID N° 19),
d. du marqueur ARG1 (SEQ ID N° 8, SEQ ID N° 9 & SEQ ID N° 10), s'il est sélectionné, est déterminée par la présence d'au moins un peptide signature choisi parmi DVDPGEHYILK (SEQ ID N° 25), TIGIIGAPFSK (SEQ ID N° 24) ou EGLYITEEIYK (SEQ ID N° 26),
e. du marqueur BHMT (SEQ ID N° 5, SEQ ID N° 6 & SEQ ID N° 7), s'il est sélectionné, est déterminée par la présence d'au moins un peptide signature choisi parmi EATTEQQLK (SEQ ID N° 21), AIAEELAPER (SEQ ID N° 23) ou EAYNLGVR (SEQ ID N° 22),
f. du marqueur BHMT1 (SEQ ID N° 5), s'il est sélectionné, est déterminée par la présence du peptide signature EATTEQQLK (SEQ ID N° 21),
g. de la combinaison des marqueurs BHMT1 (SEQ ID N°5) et BHMT2 (SEQ ID N° 6 & SEQ ID N° 7), si elle est sélectionnée, est déterminée par la présence d'au moins un peptide signature choisi parmi AIAEELAPER (SEQ ID N° 23) ou EAYNLGVR (SEQ ID N° 22),
h. du marqueur FABP1 (SEQ ID N° 11), s'il est sélectionné, est déterminée par la présence d'au moins un peptide signature choisi parmi AIGLPEELIQK (SEQ ID N° 27), FTITAGSK (SEQ ID N° 28) ou TVVQLEGDNK (SEQ ID N° 29),
i. du marqueur GSTA1 (SEQ ID N° 12), s'il est sélectionné, est déterminée par la présence du peptide signature LHYFNAR (SEQ ID N° 30),
j. du marqueur ALT1 (SEQ ID N° 13), s'il est sélectionné, est déterminée par la présence d'au moins un peptide signature choisi parmi ALELEQELR (SEQ ID N° 34), LLVAGEGHTR (SEQ ID N° 35) ou KPFTEVIR (SEQ ID N° 36), sachant que ALT1 (SEQ ID N° 13), s'il est sélectionné, est plus particulièrement déterminée par la présence du peptide signature ALELEQELR (SEQ ID N° 34) ou LLVAGEGHTR (SEQ ID N° 35).

10. Procédé *in vitro* selon l'une quelconque des revendications précédentes dans lequel on dose au moins un des marqueurs (dit « marqueur sélectionné ») tels que défini à l'une quelconque des revendications 1 à 6, dont au moins un marqueur ADH1B (SEQ ID N° 2) et/ou la combinaison des marqueurs ADH1B (SEQ ID N° 2) et ADH1A (SEQ ID N° 1) à partir d'un échantillon sanguin prélevé sur un individu à un temps t₀, lequel procédé comprend les étapes suivantes :
a) l'ajout d'une quantité connue d'une protéine isotopiquement marquée homologue du marqueur sélectionné dans l'échantillon sanguin,
b) le traitement de l'échantillon sanguin pour extraire au moins une partie des protéines abondantes ou au moins une partie des glycoprotéines,
c) le traitement de l'échantillon après extraction d'au moins une partie des protéines abondantes ou des glycoprotéines pour générer des peptides protéolytiques, de préférence par digestion, étant entendu que parmi les peptides protéolytiques obtenus se trouvent les peptides signatures des marqueurs sélectionnés tels que défini à l'une quelconque des revendications 7 à 9,
d) le dosage quantitatif par spectrométrie de masse d'au moins un peptide signature généré à partir du marqueur sélectionné,
e) la détermination d'un rapport de l'abondance du peptide signature isotopiquement marqué, ajouté à l'étape a), sur l'abondance du peptide signature non marqué issu de l'échantillon sanguin,
f) le calcul à partir du rapport obtenu à l'étape e) et de la quantité connue de ladite protéine isotopiquement marquée ajoutée à l'étape a), de la concentration dudit marqueur sélectionné dans l'échantillon.

11. Procédé *in vitro* selon la revendication 10, **caractérisé en ce que** l'étape b) est effectuée par déplétion et préférentiellement par glycodéplétion.

12. Procédé *in vitro* selon la revendication 10 ou 11, **caractérisé en ce que** le procédé est effectué à un temps t₀ et à un temps t₁ supérieur à t₀, et comprend après l'étape f), la comparaison de la concentration du marqueur sélectionné dans l'échantillon entre t₁ et t₀.

13. Procédé *in vitro* selon les revendications 10 à 12, **caractérisé en ce que** l'étape c) est effectuée par digestion enzymatique, de préférence par l'emploi de trypsine ou d'un mélange d'EndolysC et de trypsine.

14. Procédé *in vitro* selon l'une quelconque des revendications 1 à 13, **caractérisé en ce que** le trouble hépatique est une insuffisance hépatique aiguë ou une stéatohépatite non-alcoolique.

15. Procédé *in vitro* pour évaluer la toxicité hépatique d'une molécule, comprenant le dosage d'au moins un des marqueurs (dit « marqueur sélectionné ») tels que défini à l'une quelconque des revendications 1 à 6, dont au moins un marqueur ADH1B (SEQ ID N° 2) et/ou la combinaison des marqueurs ADH1B (SEQ ID N° 2) et ADH1A (SEQ ID N° 1) à partir d'un échantillon sanguin issu d'un individu auquel ladite molécule a été préalablement administrée, selon les étapes a) à f) du procédé selon l'une quelconque des revendications 10 à 13.

16. Procédé *in vitro* selon l'une quelconque des revendications 1 à 15, **caractérisé en ce que** l'échantillon sanguin est sélectionné dans le groupe constitué par le sang, le plasma et le sérum.

## Patentansprüche

1. Verfahren zur in-vitro-Diagnose, Überwachung, Prognose und/oder Theranostik von Lebererkrankungen, gewählt aus akutem Leberversagen, Lebersteatose und Steatohepatitis, basierend auf einer Blutprobe eines Individuums, wobei das Vorliegen und/oder die Konzentration des Markers ADH1B (SEQ ID NO: 2) und/oder das Vorliegen und/oder die Konzentration der Kombination der Marker ADH1B (SEQ ID NO: 2) und ADH1A (SEQ ID NO: 1) bestimmt werden.

2. In-vitro-Verfahren nach Anspruch 1, wobei das Vorliegen und/oder die Konzentration von wenigstens einem zusätzlichen Marker, bevorzugt wenigstens zwei zusätzlichen Markern und noch stärker bevorzugt wenigstens drei zusätzlichen Markern bestimmt werden, wobei die Marker gewählt sind aus der Gruppe, umfassend: ADH4 (SEQ ID NO: 3 & SEQ ID NO: 4), BHMT (SEQ ID NO: 5, SEQ ID NO: 6 & SEQ ID NO: 7), ARG1 (SEQ ID NO: 8, SEQ ID NO: 9 & SEQ ID NO: 10), FABP1 (SEQ ID NO: 11), GSTA1 (SEQ ID NO: 12) und ALT1 (SEQ ID NO: 13).

3. In-vitro-Verfahren nach einem der Ansprüche 1 bis 2, wobei wenigstens das Vorliegen und/oder die Konzentration wenigstens eines zusätzlichen Markers, gewählt aus ADH4 (SEQ ID NO: 3 & SEQ ID NO: 4) und BHMT (SEQ ID NO: 5, SEQ ID NO: 6 & SEQ ID NO: 7), bestimmt werden.

4. In-vitro-Verfahren nach einem der Ansprüche 1 bis 3, **dadurch gekennzeichnet, dass** wenigstens das Vorliegen und/oder die Konzentration des Markers ADH1B (SEQ ID NO: 2) oder der Kombination der Marker ADH1B (SEQ ID NO: 2) und ADH1A (SEQ ID NO: 1) einerseits und der Marker ADH4 (SEQ ID NO: 3 & SEQ ID NO: 4) und BHMT (SEQ ID NO: 5, SEQ ID NO: 6 & SEQ ID NO: 7) andererseits bestimmt werden.

5. In-vitro-Verfahren nach einem der Ansprüche 2 bis 4, **dadurch gekennzeichnet, dass** der Marker BHMT und die Sequenzvariante BHMT1 (SEQ ID NO: 5) und/oder die Kombination der Sequenzvarianten BHMT1 (SEQ ID NO: 5) und BHMT2 (SEQ ID NO: 6 & SEQ ID NO: 7) ist.

6. In-vitro-Verfahren nach einem der Ansprüche 1 bis 5, **dadurch gekennzeichnet, dass** zusätzlich das Vorliegen und/oder die Konzentration des Markers CK18 bestimmt werden.

7. In-vitro-Verfahren nach einem der Ansprüche 1 bis 6, **dadurch gekennzeichnet, dass** für die Bestimmung des Vorliegens und/oder Konzentration der Marker in den Markern vorhandene Peptidsignaturen verwendet werden.

8. In vitro-Verfahren nach Anspruch 7, wobei die Signaturpeptide ausgehend von den Markern durch einen, bevorzugt enzymatischen, Verdauungsprozess erzeugt werden.

9. In vitro-Verfahren nach Anspruch 7 oder 8, wobei das Vorliegen und/oder die Konzentration:
a. der Kombination der Marker ADH1A (SEQ ID NO: 1) und ADH1B (SEQ ID NO: 2), falls gewählt, bestimmt wird durch das Vorliegen wenigstens eines Signaturpeptids, gewählt aus INEGFDLLHSGK (SEQ ID NO: 16) und FSLDALITHVLPFEK (SEQ ID NO: 15),
b. des Markers ADH1B (SEQ ID NO: 2), falls gewählt, bestimmt wird durch das Vorliegen des Signaturpeptids AAVLWEVK (SEQ ID NO: 17),
c. des Markers ADH4 (SEQ ID NO: 3 & SEQ ID NO: 4), falls gewählt, bestimmt wird durch das Vorliegen wenigstens eines Signaturpeptids, gewählt aus FNLDALVTHTLPFDK (SEQ ID NO: 20), IDDDANLER (SEQ ID NO: 18) oder IIGIDINSEK (SEQ ID NO: 19),
d. des Markers ARG1 (SEQ ID NO: 8, SEQ ID NO: 9 & SEQ ID NO: 10), falls gewählt, bestimmt wird durch das Vorliegen wenigstens eines Signaturpeptids, gewählt aus DVDPGEHYILK (SEQ ID NO: 25), TIGIIGAPFSK (SEQ ID NO: 24) oder EGLYITEEIYK (SEQ ID NO: 26),
e. des Markers BHMT (SEQ ID NO: 5, SEQ ID NO: 6 & SEQ ID NO: 7), falls gewählt, bestimmt wird durch das Vorliegen wenigstens eines Signaturpeptids, gewählt aus EATTEQQLK (SEQ ID NO: 21), AIAEELAPER (SEQ ID NO: 23) oder EAYNLGVR (SEQ ID NO: 22),
f. des Markers BHMT1 (SEQ ID NO: 5), falls gewählt, bestimmt wird durch das Vorliegen des Signaturpeptids EATTEQQLK (SEQ ID NO: 21),
g. der Kombination der Marker BHMT1 (SEQ ID NO: 5) und BHMT2 (SEQ ID NO: 6 & SEQ ID NO: 7), falls gewählt, bestimmt wird durch das Vorliegen wenigstens eines Signaturpeptids, gewählt aus AIAEELAPER (SEQ ID NO: 23) oder EAYNLGVR (SEQ ID NO: 22),
h. des Markers FABP1 (SEQ ID NO: 11), falls gewählt, bestimmt wird durch das Vorliegen wenigstens eines Signaturpeptids, gewählt aus AIGLPEELIQK (SEQ ID NO: 27), FTITAGSK (SEQ ID NO: 28) oder TVVQLEGDNK (SEQ ID NO: 29),
i. des Markers GSTA1 (SEQ ID NO: 12), falls gewählt, bestimmt wird durch das Vorliegen des Signaturpeptids LHYFNAR (SEQ ID NO: 30),
j. des Markers ALT1 (SEQ ID NO: 13), falls gewählt, bestimmt wird durch das Vorliegen wenigstens eines Signaturpeptids, gewählt aus ALELEQELR (SEQ ID NO: 34), LLVAGEGHTR (SEQ ID NO: 35) oder KPFTEVIR (SEQ ID NO: 36), wobei ALT1 (SEQ ID NO: 13), falls gewählt, insbesondere durch das Vorliegen des Signaturpeptids ALELEQELR (SEQ ID NO: 34) oder LLVAGEGHTR (SEQ ID NO: 35) bestimmt wird.

10. In-vitro-Verfahren nach einem der vorstehenden Ansprüche, wobei wenigstens einer der Marker (als "gewählter Marker" bezeichnet), wie er in einem der Ansprüche 1 bis 6 definiert ist, einschließlich wenigstens eines Markers ADH1B (SEQ ID NO: 2) und/oder der Kombination der Marker ADH1B (SEQ ID NO: 2) und ADH1A (SEQ ID NO: 1) basierend auf einer Blutprobe, die einem Individuum zu einem Zeitpunkt t₀ entnommen wurde, bestimmt wird, wobei das Verfahren die folgenden Schritte umfasst:
a. Zugabe einer bekannten Menge eines isotopisch markierten Proteins, das zu dem aus der Blutprobe gewählten Marker homolog ist,
b. Behandlung der Blutprobe zur Extraktion wenigstens eines Teils der in großer Menge vorhandenen Proteine oder wenigstens eines Teils der Glykoproteine,
c. Behandlung der Probe nach der Extraktion wenigstens eines Teils der in großer Menge vorhandenen Proteine oder Glykoproteine zur Erzeugung proteolytischer Peptide, bevorzugt durch Verdauung, wobei vorausgesetzt wird, dass sich unter den erhaltenen proteolytischen Peptiden die Signaturpeptide der gewählten Marker gemäß einem der Ansprüche 7 bis 9 befinden,
d. quantitative Bestimmung wenigstens eines ausgehend von dem gewählten Marker erzeugten Signaturpeptids mittels Massenspektrometrie,
e. Bestimmung eines Verhältnisses der Häufigkeit des in Schritt a) zugegebenen isotopisch markierten Signaturpeptids zur Häufigkeit des unmarkierten Signaturpeptids aus der Blutprobe,
f. Berechnung der Konzentration des in der Probe gewählten Markers aus dem in Schritt e) erhaltenen Verhältnis und der bekannten Menge des in Schritt a) zugegebenen isotopisch markierten Proteins.

11. In-vitro-Verfahren nach Anspruch 10, **dadurch gekennzeichnet, dass** Schritt b) durch Depletion und bevorzugt durch Glykodepletion durchgeführt wird.

12. In-vitro-Verfahren nach Anspruch 10 oder 11, **dadurch gekennzeichnet, dass** das Verfahren zu einem Zeitpunkt tₒ und zu einem Zeitpunkt t₁ größer als tₒ durchgeführt wird und nach Schritt f) den Vergleich der Konzentration des gewählten Markers in der Probe zwischen t₁ und tₒ umfasst.

13. In-vitro-Verfahren nach den Ansprüchen 10 bis 12, **dadurch gekennzeichnet, dass** Schritt c) durch enzymatische Verdauung, bevorzugt unter Verwendung von Trypsin oder einer Mischung aus EndolysC und Trypsin, durchgeführt wird.

14. In-vitro-Verfahren nach einem der Ansprüche 1 bis 13, **dadurch gekennzeichnet, dass** die Lebererkrankung ein akutes Leberversagen oder eine nicht-alkoholische Steatohepatitis ist.

15. In-vitro-Verfahren zur Bewertung der hepatischen Toxizität eines Moleküls, umfassend die quantitative Bestimmung wenigstens eines der Marker (als "gewählter Marker" bezeichnet) wie in einem der Ansprüche 1 bis 6 definiert, darunter wenigstens ein Marker ADH1B (SEQ ID NO: 2) und/oder die Kombination der Marker ADH1B (SEQ ID NO: 2) und ADH1A (SEQ ID NO: 1) aus einer Blutprobe eines Individuums, dem dieses Molekül zuvor verabreicht wurde, gemäß den Schritten a) bis f) des Verfahrens nach einem der Ansprüche 10 bis 13.

16. In-vitro-Verfahren nach einem der Ansprüche 1 bis 15, **dadurch gekennzeichnet, dass** die Blutprobe aus der Gruppe, umfassend Blut, Plasma und Serum gewählt wird.

## Claims

1. Process for in vitro diagnosis, monitoring, prognosis and/or theranostics of hepatic disorders chosen from acute liver injury, hepatic steatosis and steatohepatitis, from a blood sample originating from a subject, in which process the presence and/or concentration of the marker ADH1B (SEQ ID NO. 2) and/or the presence and/or concentration of the combination of the markers ADH1B (SEQ ID NO. 2) and ADH1A (SEQ ID NO. 1) is determined.

2. In vitro process according to claim 1 wherein the presence and/or concentration of at least one additional marker, preferentially of at least two additional markers, and even more preferentially of at least three additional markers is determined, said markers being selected from the group consisting of: ADH4 (SEQ ID NO. 3 & SEQ ID NO. 4), BHMT (SEQ ID NO. 5, SEQ ID NO. 6 & SEQ ID NO. 7), ARG1 (SEQ ID NO. 8, SEQ ID NO. 9 & SEQ ID NO. 10), FABP1 (SEQ ID NO. 11), GSTA1 (SEQ ID NO. 12) and ALT1 (SEQ ID NO. 13).

3. In vitro process according to any one of claims 1 to 2, wherein at least the presence and/or concentration of at least one additional marker selected from ADH4 (SEQ ID NO. 3 & SEQ ID NO. 4) and BHMT (SEQ ID NO. 5, SEQ ID NO. 6 & SEQ ID NO. 7) is determined.

4. In vitro process according to any one of claims 1 to 3, wherein at least the presence and/or concentration of the marker ADH1B (SEQ ID NO. 2) or of the combination of the markers ADH1B (SEQ ID NO. 2) and ADH1A (SEQ ID NO. 1), on one hand, and of the markers ADH4 (SEQ ID NO. 3 & SEQ ID NO. 4) and BHMT (SEQ ID NO. 5, SEQ ID NO. 6 & SEQ ID NO. 7), on the other, is determined.

5. In vitro process according to any one of claims 2 to 4 **characterised in that** the marker BHMT is the sequence variant BHMT1 (SEQ ID NO. 5) and/or the combination of the sequence variants BHMT1 (SEQ ID NO. 5) and BHMT2 (SEQ ID NO. 6 & SEQ ID NO. 7).

6. In vitro process according to any one of claims 1 to 5, wherein the presence and/or concentration of the marker CK18 is further determined.

7. In vitro process according to any one of claims 1 to 6, wherein signature peptides present in said markers are used for said determination of the presence and/or concentration of said markers.

8. In vitro process according to claim 7, wherein said signature peptides are generated from said markers by a digestion process, preferably enzymatic.

9. In vitro process according to claim 7 or 8, wherein the presence and/or concentration:
a. of the combination of the markers ADH1A (SEQ ID NO. 1) and ADH1B (SEQ ID NO. 2), if it is selected, is determined by the presence of at least one signature peptide chosen from INEGFDLLHSK (SEQ ID NO. 16) and FSLDALITHVLPFEK (SEQ ID NO. 15),
b. of the marker ADH1B (SEQ ID NO. 2), if it is selected, is determined by the presence of the signature peptide AAVLWEVK (SEQ ID NO. 17),
c. of the marker ADH4 (SEQ ID NO. 3 & SEQ ID NO. 4), if it is selected, is determined by the presence of at least one signature peptide chosen from FNLDALVTHTLPFDK (SEQ ID NO. 20), IDDDANLER (SEQ ID NO. 18) or IIGIDINSEK (SEQ ID NO. 19),
d. of the marker ARG1 (SEQ ID NO. 8, SEQ ID NO. 9 & SEQ ID NO. 10), if it is selected, is determined by the presence of at least one signature peptide chosen from DVDPGEHYILK (SEQ ID NO. 25), TIGIIGAPFSK (SEQ ID NO. 24) or EGLYITEEIYK (SEQ ID NO. 26),
e. of the marker BHMT (SEQ ID NO. 5, SEQ ID NO. 6 & SEQ ID NO. 7), if it is selected, is determined by the presence of at least one signature peptide chosen from EATTEQQLK (SEQ ID NO. 21), AIAEELAPER (SEQ ID NO. 23) or EAYNLGVR (SEQ ID NO. 22),
f. of the marker BHMT1 (SEQ ID NO. 5), if it is selected, is determined by the presence of the signature peptide EATTEQQLK (SEQ ID NO. 21),
g. of the combination of the markers BHMT1 (SEQ ID NO. 5) and BHMT2 (SEQ ID NO. 6 & SEQ ID NO. 7), if it is selected, is determined by the presence of at least one signature peptide chosen from AIAEELAPER (SEQ ID NO. 23) or EAYNLGVR (SEQ ID NO. 22),
h. of the marker FABP1 (SEQ ID NO. 11), if it is selected, is determined by the presence of at least one signature peptide chosen from AIGLPEELIQK (SEQ ID NO. 27), FTITAGSK (SEQ ID NO. 28) or TVVQLEGDNK (SEQ ID NO. 29),
i. of the marker GSTA1 (SEQ ID NO. 12), if it is selected, is determined by the presence of the signature peptide LHYFNAR (SEQ ID NO. 30),
j. of the marker ALT1 (SEQ ID NO. 13), if it is selected, is determined by the presence of at least one signature peptide chosen from ALELEQELR (SEQ ID NO. 34), LLVAGEGHTR (SEQ ID NO. 35) or KPFTEVIR (SEQ ID NO. 36) providing that ALT1 (SEQ ID NO. 13), if it is selected, is more particularly determined by the presence of the signature peptide ALELEQELR (SEQ ID NO. 34) or LLVAGEGHTR (SEQ ID NO. 35).

10. In vitro process according to any one of the preceding claims, wherein at least one of the markers (referred to as "selected marker") as defined in any one of claims 1 to 6 including at least one marker ADH1B (SEQ ID NO. 2) and/or of the combination of the markers ADH1B (SEQ ID NO. 2) and ADH1A (SEQ ID NO. 1), is assayed from a blood sample taken from a subject at the time t₀, said process comprising the following steps:
a) adding a known quantity of an isotopically labelled homologous protein (referred to as "standard") of the selected marker to the blood sample,
b) treating the blood sample to extract at least a portion of the abundant proteins or at least a portion of the glycoproteins,
c) treating the sample after extracting at least a portion of the abundant proteins or glycoproteins to generate proteolytic peptides, preferably by digestion, it being understood that the proteolytic proteins obtained include the signature peptides of the selected markers as defined in any one of claims 7 to 9,
d) quantitative assay by mass spectrometry of at least one signature peptide generated from the selected marker,
e) determining a ratio of the abundance of the isotopically labelled signature peptide, from the standard, added in step a), to the abundance of the non-labelled signature peptide from the blood sample,
f) computing the concentration of said selected marker in the sample based on the ratio obtained in step e) and on the known quantity of said isotopically labelled protein added in step a).

11. In vitro process according to claim 10, **characterised in that** step b) is performed by depletion and preferentially by glycodepletion.

12. In vitro process according to claim 10 or 11, **characterised in that** the process is performed at a time t₀ and at a time t₁ greater than t₀, and comprises after step f) the comparison of the concentration of the selected marker in the sample between t₁ and t₀.

13. In vitro process according to claims 10 to 12, **characterised in that** step c) is performed by enzymatic digestion, preferably by using trypsin or a mixture of EndolysC and trypsin.

14. In vitro process according to any one of claims 1 to 13, **characterised in that** the liver disorder is acute liver injury or non-alcoholic steatohepatitis.

15. Process in vitro for assessing the toxicity of a molecule, comprising assaying at least one of the markers (referred to as "selected marker") as defined in any one of claims 1 to 6 including at least one marker ADH1B (SEQ ID NO. 2) and/or of the combination of the markers ADH1B (SEQ ID NO. 2) and ADH1A (SEQ ID NO. 1), from a blood sample from a subject previously administered with said molecule, according to steps a) to f) of the process according to any one of claims 10 to 13.

16. Process in vitro according to any one of claims 1 to 15, **characterised in that** the blood sample is selected from the group consisting of: blood, plasma and serum.


### SEQUENCE LISTING

<110> Commissariat à l'énergie atomique et aux énergies alternatives
<120> Procédé de diagnostic in vitro d'atteintes hépatiques
<130> BCT170014 QT
<160> 36
<170> PatentIn version 3.5
<210> 1
   <211> 375
   <212> PRT
   <213> Homo sapiens
<400> 1
   Met Ser Thr Ala Gly Lys Val Ile Lys Cys Lys Ala Ala Val Leu Trp
   1               5                   10                  15
   Glu Leu Lys Lys Pro Phe Ser Ile Glu Glu Val Glu Val Ala Pro Pro
               20                  25                  30
   Lys Ala His Glu Val Arg Ile Lys Met Val Ala Val Gly Ile Cys Gly
           35                  40                  45
   Thr Asp Asp His Val Val Ser Gly Thr Met Val Thr Pro Leu Pro Val
       50                  55                  60
   Ile Leu Gly His Glu Ala Ala Gly Ile Val Glu Ser Val Gly Glu Gly
   65                  70                  75                  80
   Val Thr Thr Val Lys Pro Gly Asp Lys Val Ile Pro Leu Ala Ile Pro
                   85                  90                  95
   Gln Cys Gly Lys Cys Arg Ile Cys Lys Asn Pro Glu Ser Asn Tyr Cys
               100                 105                 110
   Leu Lys Asn Asp Val Ser Asn Pro Gln Gly Thr Leu Gln Asp Gly Thr
           115                 120                 125
   Ser Arg Phe Thr Cys Arg Arg Lys Pro Ile His His Phe Leu Gly Ile
       130                 135                 140
   Ser Thr Phe Ser Gln Tyr Thr Val Val Asp Glu Asn Ala Val Ala Lys
   145                 150                 155                 160
   Ile Asp Ala Ala Ser Pro Leu Glu Lys Val Cys Leu Ile Gly Cys Gly
                   165                 170                 175
   Phe Ser Thr Gly Tyr Gly Ser Ala Val Asn Val Ala Lys Val Thr Pro
   180 185 190
   Gly Ser Thr Cys Ala Val Phe Gly Leu Gly Gly Val Gly Leu Ser Ala
   195 200 205
   Ile Met Gly Cys Lys Ala Ala Gly Ala Ala Arg Ile Ile Ala Val Asp
   210 215 220
   Ile Asn Lys Asp Lys Phe Ala Lys Ala Lys Glu Leu Gly Ala Thr Glu
   225 230 235 240
   Cys Ile Asn Pro Gln Asp Tyr Lys Lys Pro Ile Gln Glu Val Leu Lys
   245 250 255
   Glu Met Thr Asp Gly Gly Val Asp Phe Ser Phe Glu Val Ile Gly Arg
   260 265 270
   Leu Asp Thr Met Met Ala Ser Leu Leu Cys Cys His Glu Ala Cys Gly
   275 280 285
   Thr Ser Val Ile Val Gly Val Pro Pro Asp Ser Gln Asn Leu Ser Met
   290 295 300
   Asn Pro Met Leu Leu Leu Thr Gly Arg Thr Trp Lys Gly Ala Ile Leu
   305 310 315 320
   Gly Gly Phe Lys Ser Lys Glu Cys Val Pro Lys Leu Val Ala Asp Phe
   325 330 335
   Met Ala Lys Lys Phe Ser Leu Asp Ala Leu Ile Thr His Val Leu Pro
   340 345 350
   Phe Glu Lys Ile Asn Glu Gly Phe Asp Leu Leu His Ser Gly Lys Ser
   355 360 365
   Ile Arg Thr Ile Leu Met Phe
   370 375
<210> 2
   <211> 375
   <212> PRT
   <213> Homo sapiens
<400> 2
<210> 3
   <211> 380
   <212> PRT
   <213> Homo sapiens
<400> 3
   Met Gly Thr Lys Gly Lys Val Ile Lys Cys Lys Ala Ala Ile Ala Trp
   1               5                   10                  15
   Glu Ala Gly Lys Pro Leu Cys Ile Glu Glu Val Glu Val Ala Pro Pro
               20                  25                  30
   Lys Ala His Glu Val Arg Ile Gln Ile Ile Ala Thr Ser Leu Cys His
           35                  40                  45
   Thr Asp Ala Thr Val Ile Asp Ser Lys Phe Glu Gly Leu Ala Phe Pro
       50                  55                  60
   Val Ile Val Gly His Glu Ala Ala Gly Ile Val Glu Ser Ile Gly Pro
   65                  70                  75                  80
   Gly Val Thr Asn Val Lys Pro Gly Asp Lys Val Ile Pro Leu Tyr Ala
                   85                  90                  95
   Pro Leu Cys Arg Lys Cys Lys Phe Cys Leu Ser Pro Leu Thr Asn Leu
               100                 105                 110
   Cys Gly Lys Ile Ser Asn Leu Lys Ser Pro Ala Ser Asp Gln Gln Leu
   115 120 125
   Met Glu Asp Lys Thr Ser Arg Phe Thr Cys Lys Gly Lys Pro Val Tyr
   130 135 140
   His Phe Phe Gly Thr Ser Thr Phe Ser Gln Tyr Thr Val Val Ser Asp
   145 150 155 160
   Ile Asn Leu Ala Lys Ile Asp Asp Asp Ala Asn Leu Glu Arg Val Cys
   165 170 175
   Leu Leu Gly Cys Gly Phe Ser Thr Gly Tyr Gly Ala Ala Ile Asn Asn
   180 185 190
   Ala Lys Val Thr Pro Gly Ser Thr Cys Ala Val Phe Gly Leu Gly Gly
   195 200 205
   Val Gly Leu Ser Ala Val Met Gly Cys Lys Ala Ala Gly Ala Ser Arg
   210 215 220
   Ile Ile Gly Ile Asp Ile Asn Ser Glu Lys Phe Val Lys Ala Lys Ala
   225 230 235 240
   Leu Gly Ala Thr Asp Cys Leu Asn Pro Arg Asp Leu His Lys Pro Ile
   245 250 255
   Gln Glu Val Ile Ile Glu Leu Thr Lys Gly Gly Val Asp Phe Ala Leu
   260 265 270
   Asp Cys Ala Gly Gly Ser Glu Thr Met Lys Ala Ala Leu Asp Cys Thr
   275 280 285
   Thr Ala Gly Trp Gly Ser Cys Thr Phe Ile Gly Val Ala Ala Gly Ser
   290 295 300
   Lys Gly Leu Thr Ile Phe Pro Glu Glu Leu Ile Ile Gly Arg Thr Ile
   305 310 315 320
   Asn Gly Thr Phe Phe Gly Gly Trp Lys Ser Val Asp Ser Ile Pro Lys
   325 330 335
   Leu Val Thr Asp Tyr Lys Asn Lys Lys Phe Asn Leu Asp Ala Leu Val
   340 345 350
   Thr His Thr Leu Pro Phe Asp Lys Ile Ser Glu Ala Phe Asp Leu Met
   355 360 365
   Asn Gln Gly Lys Ser Val Arg Thr Ile Leu Ile Phe
   370 375 380
<210> 4
   <211> 399
   <212> PRT
   <213> Homo sapiens
<400> 4
   Met Leu Val Arg Gly Pro His Phe Glu Leu Gln Arg Cys Lys Thr His
   1               5                   10                  15
   Leu Phe Ser Ser Asn Tyr Leu Thr Gln Val Ile Lys Cys Lys Ala Ala
               20                  25                  30
   Ile Ala Trp Glu Ala Gly Lys Pro Leu Cys Ile Glu Glu Val Glu Val
           35                  40                  45
   Ala Pro Pro Lys Ala His Glu Val Arg Ile Gln Ile Ile Ala Thr Ser
       50                  55                  60
   Leu Cys His Thr Asp Ala Thr Val Ile Asp Ser Lys Phe Glu Gly Leu
   65                  70                  75                  80
   Ala Phe Pro Val Ile Val Gly His Glu Ala Ala Gly Ile Val Glu Ser
                   85                  90                  95
   Ile Gly Pro Gly Val Thr Asn Val Lys Pro Gly Asp Lys Val Ile Pro
               100                 105                 110
   Leu Tyr Ala Pro Leu Cys Arg Lys Cys Lys Phe Cys Leu Ser Pro Leu
           115                 120                 125
   Thr Asn Leu Cys Gly Lys Ile Ser Asn Leu Lys Ser Pro Ala Ser Asp
       130                 135                 140
   Gln Gln Leu Met Glu Asp Lys Thr Ser Arg Phe Thr Cys Lys Gly Lys
   145                 150                 155                 160
   Pro Val Tyr His Phe Phe Gly Thr Ser Thr Phe Ser Gln Tyr Thr Val
                   165                 170                 175
   Val Ser Asp Ile Asn Leu Ala Lys Ile Asp Asp Asp Ala Asn Leu Glu
               180                 185                 190
   Arg Val Cys Leu Leu Gly Cys Gly Phe Ser Thr Gly Tyr Gly Ala Ala
               195                 200                 205
   Ile Asn Asn Ala Lys Val Thr Pro Gly Ser Thr Cys Ala Val Phe Gly
       210                 215                 220
   Leu Gly Gly Val Gly Leu Ser Ala Val Met Gly Cys Lys Ala Ala Gly
   225                 230                 235                 240
   Ala Ser Arg Ile Ile Gly Ile Asp Ile Asn Ser Glu Lys Phe Val Lys
                   245                 250                 255
   Ala Lys Ala Leu Gly Ala Thr Asp Cys Leu Asn Pro Arg Asp Leu His
               260                 265                 270
   Lys Pro Ile Gln Glu Val Ile Ile Glu Leu Thr Lys Gly Gly Val Asp
           275                 280                 285
   Phe Ala Leu Asp Cys Ala Gly Gly Ser Glu Thr Met Lys Ala Ala Leu
       290                 295                 300
   Asp Cys Thr Thr Ala Gly Trp Gly Ser Cys Thr Phe Ile Gly Val Ala
   305                 310                 315                 320
   Ala Gly Ser Lys Gly Leu Thr Ile Phe Pro Glu Glu Leu Ile Ile Gly
                   325                 330                 335
   Arg Thr Ile Asn Gly Thr Phe Phe Gly Gly Trp Lys Ser Val Asp Ser
               340                 345                 350
   Ile Pro Lys Leu Val Thr Asp Tyr Lys Asn Lys Lys Phe Asn Leu Asp
           355                 360                 365
   Ala Leu Val Thr His Thr Leu Pro Phe Asp Lys Ile Ser Glu Ala Phe
       370                 375                 380
   Asp Leu Met Asn Gln Gly Lys Ser Val Arg Thr Ile Leu Ile Phe
   385                 390                 395
<210> 5
   <211> 406
   <212> PRT
   <213> Homo sapiens
<400> 5
   Met Pro Pro Val Gly Gly Lys Lys Ala Lys Lys Gly Ile Leu Glu Arg
   1               5                   10                  15
   Leu Asn Ala Gly Glu Ile Val Ile Gly Asp Gly Gly Phe Val Phe Ala
               20                  25                  30
   Leu Glu Lys Arg Gly Tyr Val Lys Ala Gly Pro Trp Thr Pro Glu Ala
           35                  40                  45
   Ala Val Glu His Pro Glu Ala Val Arg Gln Leu His Arg Glu Phe Leu
       50                  55                  60
   Arg Ala Gly Ser Asn Val Met Gln Thr Phe Thr Phe Tyr Ala Ser Glu
   65                  70                  75                  80
   Asp Lys Leu Glu Asn Arg Gly Asn Tyr Val Leu Glu Lys Ile Ser Gly
                   85                  90                  95
   Gln Glu Val Asn Glu Ala Ala Cys Asp Ile Ala Arg Gln Val Ala Asp
               100                 105                 110
   Glu Gly Asp Ala Leu Val Ala Gly Gly Val Ser Gln Thr Pro Ser Tyr
           115                 120                 125
   Leu Ser Cys Lys Ser Glu Thr Glu Val Lys Lys Val Phe Leu Gln Gln
       130                 135                 140
   Leu Glu Val Phe Met Lys Lys Asn Val Asp Phe Leu Ile Ala Glu Tyr
   145                 150                 155                 160
   Phe Glu His Val Glu Glu Ala Val Trp Ala Val Glu Thr Leu Ile Ala
                   165                 170                 175
   Ser Gly Lys Pro Val Ala Ala Thr Met Cys Ile Gly Pro Glu Gly Asp
               180                 185                 190
   Leu His Gly Val Pro Pro Gly Glu Cys Ala Val Arg Leu Val Lys Ala
           195                 200                 205
   Gly Ala Ser Ile Ile Gly Val Asn Cys His Phe Asp Pro Thr Ile Ser
       210                 215                 220
   Leu Lys Thr Val Lys Leu Met Lys Glu Gly Leu Glu Ala Ala Arg Leu
   225                 230                 235                 240
   Lys Ala His Leu Met Ser Gln Pro Leu Ala Tyr His Thr Pro Asp Cys
                   245                 250                 255
   Asn Lys Gln Gly Phe Ile Asp Leu Pro Glu Phe Pro Phe Gly Leu Glu
               260                 265                 270
   Pro Arg Val Ala Thr Arg Trp Asp Ile Gln Lys Tyr Ala Arg Glu Ala
   275 280 285
   Tyr Asn Leu Gly Val Arg Tyr Ile Gly Gly Cys Cys Gly Phe Glu Pro
   290 295 300
   Tyr His Ile Arg Ala Ile Ala Glu Glu Leu Ala Pro Glu Arg Gly Phe
   305 310 315 320
   Leu Pro Pro Ala Ser Glu Lys His Gly Ser Trp Gly Ser Gly Leu Asp
   325 330 335
   Met His Thr Lys Pro Trp Val Arg Ala Arg Ala Arg Lys Glu Tyr Trp
   340 345 350
   Glu Asn Leu Arg Ile Ala Ser Gly Arg Pro Tyr Asn Pro Ser Met Ser
   355 360 365
   Lys Pro Asp Gly Trp Gly Val Thr Lys Gly Thr Ala Glu Leu Met Gln
   370 375 380
   Gln Lys Glu Ala Thr Thr Glu Gln Gln Leu Lys Glu Leu Phe Glu Lys
   385 390 395 400
   Gln Lys Phe Lys Ser Gln
   405
<210> 6
   <211> 363
   <212> PRT
   <213> Homo sapiens
<400> 6
   Met Ala Pro Ala Gly Arg Pro Gly Ala Lys Lys Gly Ile Leu Glu Arg
   1 5 10 15
   Leu Glu Ser Gly Glu Val Val Ile Gly Asp Gly Ser Phe Leu Ile Thr
   20 25 30
   Leu Glu Lys Arg Gly Tyr Val Lys Ala Gly Leu Trp Thr Pro Glu Ala
   35 40 45
   Val Ile Glu His Pro Asp Ala Val Arg Gln Leu His Met Glu Phe Leu
   50 55 60
   Arg Ala Gly Ser Asn Val Met Gln Thr Phe Thr Phe Ser Ala Ser Glu
   65 70 75 80
   Asp Asn Met Glu Ser Lys Trp Glu Asp Val Asn Ala Ala Ala Cys Asp
                   85                  90                  95
   Leu Ala Arg Glu Val Ala Gly Lys Gly Asp Ala Leu Val Ala Gly Gly
               100                 105                 110
   Ile Cys Gln Thr Ser Ile Tyr Lys Tyr Gln Lys Asp Glu Ala Arg Ile
           115                 120                 125
   Lys Lys Leu Phe Arg Gln Gln Leu Glu Val Phe Ala Trp Lys Asn Val
       130                 135                 140
   Asp Phe Leu Ile Ala Glu Tyr Phe Glu His Val Glu Glu Ala Val Trp
   145                 150                 155                 160
   Ala Val Glu Val Leu Lys Glu Ser Asp Arg Pro Val Ala Val Thr Met
                   165                 170                 175
   Cys Ile Gly Pro Glu Gly Asp Met His Asp Ile Thr Pro Gly Glu Cys
               180                 185                 190
   Ala Val Arg Leu Val Lys Ala Gly Ala Ser Ile Val Gly Val Asn Cys
           195                 200                 205
   Arg Phe Gly Pro Asp Thr Ser Leu Lys Thr Met Glu Leu Met Lys Glu
       210                 215                 220
   Gly Leu Glu Trp Ala Gly Leu Lys Ala His Leu Met Val Gln Pro Leu
   225                 230                 235                 240
   Gly Phe His Ala Pro Asp Cys Gly Lys Glu Gly Phe Val Asp Leu Pro
                   245                 250                 255
   Glu Tyr Pro Phe Gly Leu Glu Ser Arg Val Ala Thr Arg Trp Asp Ile
               260                 265                 270
   Gln Lys Tyr Ala Arg Glu Ala Tyr Asn Leu Gly Val Arg Tyr Ile Gly
           275                 280                 285
   Gly Cys Cys Gly Phe Glu Pro Tyr His Ile Arg Ala Ile Ala Glu Glu
       290                 295                 300
   Leu Ala Pro Glu Arg Gly Phe Leu Pro Pro Ala Ser Glu Lys His Gly
   305                 310                 315                 320
   Ser Trp Gly Ser Gly Leu Asp Met His Thr Lys Pro Trp Ile Arg Ala
                   325                 330                 335
   Arg Ala Arg Arg Glu Tyr Trp Glu Asn Leu Leu Pro Ala Ser Gly Arg
               340                 345                 350
   Pro Phe Cys Pro Ser Leu Ser Lys Pro Asp Phe
           355                 360
<210> 7
   <211> 299
   <212> PRT
   <213> Homo sapiens
<400> 7
   Met Ala Pro Ala Gly Arg Pro Gly Ala Lys Lys Gly Ile Leu Glu Arg
   1               5                   10                  15
   Leu Glu Ser Gly Glu Val Val Ile Gly Asp Gly Ser Phe Leu Ile Thr
               20                  25                  30
   Leu Glu Lys Arg Gly Tyr Val Lys Ala Gly Leu Trp Thr Pro Glu Ala
           35                  40                  45
   Val Ile Glu His Pro Asp Ala Val Arg Gln Leu His Met Glu Phe Leu
       50                  55                  60
   Arg Ala Gly Ser Asn Val Met Gln Thr Phe Thr Phe Ser Ala Ser Glu
   65                  70                  75                  80
   Asp Asn Met Glu Ser Lys Tyr Phe Glu His Val Glu Glu Ala Val Trp
                   85                  90                  95
   Ala Val Glu Val Leu Lys Glu Ser Asp Arg Pro Val Ala Val Thr Met
               100                 105                 110
   Cys Ile Gly Pro Glu Gly Asp Met His Asp Ile Thr Pro Gly Glu Cys
           115                 120                 125
   Ala Val Arg Leu Val Lys Ala Gly Ala Ser Ile Val Gly Val Asn Cys
       130                 135                 140
   Arg Phe Gly Pro Asp Thr Ser Leu Lys Thr Met Glu Leu Met Lys Glu
   145                 150                 155                 160
   Gly Leu Glu Trp Ala Gly Leu Lys Ala His Leu Met Val Gln Pro Leu
                   165                 170                 175
   Gly Phe His Ala Pro Asp Cys Gly Lys Glu Gly Phe Val Asp Leu Pro
               180                 185                 190
   Glu Tyr Pro Phe Gly Leu Glu Ser Arg Val Ala Thr Arg Trp Asp Ile
   195 200 205
   Gln Lys Tyr Ala Arg Glu Ala Tyr Asn Leu Gly Val Arg Tyr Ile Gly
   210 215 220
   Gly Cys Cys Gly Phe Glu Pro Tyr His Ile Arg Ala Ile Ala Glu Glu
   225 230 235 240
   Leu Ala Pro Glu Arg Gly Phe Leu Pro Pro Ala Ser Glu Lys His Gly
   245 250 255
   Ser Trp Gly Ser Gly Leu Asp Met His Thr Lys Pro Trp Ile Arg Ala
   260 265 270
   Arg Ala Arg Arg Glu Tyr Trp Glu Asn Leu Leu Pro Ala Ser Gly Arg
   275 280 285
   Pro Phe Cys Pro Ser Leu Ser Lys Pro Asp Phe
   290 295
<210> 8
   <211> 322
   <212> PRT
   <213> Homo sapiens
<400> 8
<210> 9
   <211> 330
   <212> PRT
   <213> Homo sapiens
<400> 9
   Met Ser Ala Lys Ser Arg Thr Ile Gly Ile Ile Gly Ala Pro Phe Ser
   1               5                   10                  15
   Lys Gly Gln Pro Arg Gly Gly Val Glu Glu Gly Pro Thr Val Leu Arg
               20                  25                  30
   Lys Ala Gly Leu Leu Glu Lys Leu Lys Glu Gln Val Thr Gln Asn Phe
           35                  40                  45
   Leu Ile Leu Glu Cys Asp Val Lys Asp Tyr Gly Asp Leu Pro Phe Ala
       50                  55                  60
   Asp Ile Pro Asn Asp Ser Pro Phe Gln Ile Val Lys Asn Pro Arg Ser
   65                  70                  75                  80
   Val Gly Lys Ala Ser Glu Gln Leu Ala Gly Lys Val Ala Glu Val Lys
                   85                  90                  95
   Lys Asn Gly Arg Ile Ser Leu Val Leu Gly Gly Asp His Ser Leu Ala
               100                 105                 110
   Ile Gly Ser Ile Ser Gly His Ala Arg Val His Pro Asp Leu Gly Val
           115                 120                 125
   Ile Trp Val Asp Ala His Thr Asp Ile Asn Thr Pro Leu Thr Thr Thr
       130                 135                 140
   Ser Gly Asn Leu His Gly Gln Pro Val Ser Phe Leu Leu Lys Glu Leu
   145                 150                 155                 160
   Lys Gly Lys Ile Pro Asp Val Pro Gly Phe Ser Trp Val Thr Pro Cys
                   165                 170                 175
   Ile Ser Ala Lys Asp Ile Val Tyr Ile Gly Leu Arg Asp Val Asp Pro
               180                 185                 190
   Gly Glu His Tyr Ile Leu Lys Thr Leu Gly Ile Lys Tyr Phe Ser Met
           195                 200                 205
   Thr Glu Val Asp Arg Leu Gly Ile Gly Lys Val Met Glu Glu Thr Leu
       210                 215                 220
   Ser Tyr Leu Leu Gly Arg Lys Lys Arg Pro Ile His Leu Ser Phe Asp
   225                 230                 235                 240
   Val Asp Gly Leu Asp Pro Ser Phe Thr Pro Ala Thr Gly Thr Pro Val
                   245                 250                 255
   Val Gly Gly Leu Thr Tyr Arg Glu Gly Leu Tyr Ile Thr Glu Glu Ile
   260 265 270
   Tyr Lys Thr Gly Leu Leu Ser Gly Leu Asp Ile Met Glu Val Asn Pro
   275 280 285
   Ser Leu Gly Lys Thr Pro Glu Glu Val Thr Arg Thr Val Asn Thr Ala
   290 295 300
   Val Ala Ile Thr Leu Ala Cys Phe Gly Leu Ala Arg Glu Gly Asn His
   305 310 315 320
   Lys Pro Ile Asp Tyr Leu Asn Pro Pro Lys
   325 330
<210> 10
   <211> 236
   <212> PRT
   <213> Homo sapiens
<400> 10
   Met Ser Ala Lys Ser Arg Thr Ile Gly Ile Ile Gly Ala Pro Phe Ser
   1 5 10 15
   Lys Gly Gln Pro Arg Gly Gly Val Glu Glu Gly Pro Thr Val Leu Arg
   20 25 30
   Lys Ala Gly Leu Leu Glu Lys Leu Lys Glu Gln Glu Cys Asp Val Lys
   35 40 45
   Asp Tyr Gly Asp Leu Pro Phe Ala Asp Ile Pro Asn Asp Ser Pro Phe
   50 55 60
   Gln Ile Val Lys Asn Pro Arg Ser Val Gly Lys Ala Ser Glu Gln Leu
   65 70 75 80
   Ala Gly Lys Val Ala Glu Val Lys Lys Asn Gly Arg Ile Ser Leu Val
   85 90 95
   Leu Gly Gly Asp His Ser Leu Ala Ile Gly Ser Ile Ser Gly His Ala
   100 105 110
   Arg Val His Pro Asp Leu Gly Val Ile Trp Val Asp Ala His Thr Asp
   115 120 125
   Ile Asn Thr Pro Leu Thr Thr Thr Ser Gly Asn Leu His Gly Gln Pro
   130 135 140
   Val Ser Phe Leu Leu Lys Glu Leu Lys Gly Lys Ile Pro Asp Val Pro
   145                 150                 155                 160
   Gly Phe Ser Trp Val Thr Pro Cys Ile Ser Ala Lys Asp Ile Val Tyr
                   165                 170                 175
   Ile Gly Leu Arg Asp Val Asp Pro Gly Glu His Tyr Ile Leu Lys Thr
               180                 185                 190
   Leu Gly Ile Lys Tyr Phe Ser Met Thr Glu Val Thr Arg Thr Val Asn
           195                 200                 205
   Thr Ala Val Ala Ile Thr Leu Ala Cys Phe Gly Leu Ala Arg Glu Gly
       210                 215                 220
   Asn His Lys Pro Ile Asp Tyr Leu Asn Pro Pro Lys
   225                 230                 235
<210> 11
   <211> 127
   <212> PRT
   <213> Homo sapiens
<400> 11
   Met Ser Phe Ser Gly Lys Tyr Gln Leu Gln Ser Gln Glu Asn Phe Glu
   1               5                   10                  15
   Ala Phe Met Lys Ala Ile Gly Leu Pro Glu Glu Leu Ile Gln Lys Gly
               20                  25                  30
   Lys Asp Ile Lys Gly Val Ser Glu Ile Val Gln Asn Gly Lys His Phe
           35                  40                  45
   Lys Phe Thr Ile Thr Ala Gly Ser Lys Val Ile Gln Asn Glu Phe Thr
       50                  55                  60
   Val Gly Glu Glu Cys Glu Leu Glu Thr Met Thr Gly Glu Lys Val Lys
   65                  70                  75                  80
   Thr Val Val Gln Leu Glu Gly Asp Asn Lys Leu Val Thr Thr Phe Lys
                   85                  90                  95
   Asn Ile Lys Ser Val Thr Glu Leu Asn Gly Asp Ile Ile Thr Asn Thr
               100                 105                 110
   Met Thr Leu Gly Asp Ile Val Phe Lys Arg Ile Ser Lys Arg Ile
           115                 120                 125
<210> 12
   <211> 222
   <212> PRT
   <213> Homo sapiens
<400> 12
   Met Ala Glu Lys Pro Lys Leu His Tyr Phe Asn Ala Arg Gly Arg Met
   1               5                   10                  15
   Glu Ser Thr Arg Trp Leu Leu Ala Ala Ala Gly Val Glu Phe Glu Glu
               20                  25                  30
   Lys Phe Ile Lys Ser Ala Glu Asp Leu Asp Lys Leu Arg Asn Asp Gly
           35                  40                  45
   Tyr Leu Met Phe Gln Gln Val Pro Met Val Glu Ile Asp Gly Met Lys
       50                  55                  60
   Leu Val Gln Thr Arg Ala Ile Leu Asn Tyr Ile Ala Ser Lys Tyr Asn
   65                  70                  75                  80
   Leu Tyr Gly Lys Asp Ile Lys Glu Arg Ala Leu Ile Asp Met Tyr Ile
                   85                  90                  95
   Glu Gly Ile Ala Asp Leu Gly Glu Met Ile Leu Leu Leu Pro Val Cys
               100                 105                 110
   Pro Pro Glu Glu Lys Asp Ala Lys Leu Ala Leu Ile Lys Glu Lys Ile
           115                 120                 125
   Lys Asn Arg Tyr Phe Pro Ala Phe Glu Lys Val Leu Lys Ser His Gly
       130                 135                 140
   Gln Asp Tyr Leu Val Gly Asn Lys Leu Ser Arg Ala Asp Ile His Leu
   145                 150                 155                 160
   Val Glu Leu Leu Tyr Tyr Val Glu Glu Leu Asp Ser Ser Leu Ile Ser
                   165                 170                 175
   Ser Phe Pro Leu Leu Lys Ala Leu Lys Thr Arg Ile Ser Asn Leu Pro
               180                 185                 190
   Thr Val Lys Lys Phe Leu Gln Pro Gly Ser Pro Arg Lys Pro Pro Met
           195                 200                 205
   Asp Glu Lys Ser Leu Glu Glu Ala Arg Lys Ile Phe Arg Phe
       210                 215                 220
<210> 13
   <211> 496
   <212> PRT
   <213> Homo sapiens
<400> 13
   Met Ala Ser Ser Thr Gly Asp Arg Ser Gln Ala Val Arg His Gly Leu
   1               5                   10                  15
   Arg Ala Lys Val Leu Thr Leu Asp Gly Met Asn Pro Arg Val Arg Arg
               20                  25                  30
   Val Glu Tyr Ala Val Arg Gly Pro Ile Val Gln Arg Ala Leu Glu Leu
           35                  40                  45
   Glu Gln Glu Leu Arg Gln Gly Val Lys Lys Pro Phe Thr Glu Val Ile
       50                  55                  60
   Arg Ala Asn Ile Gly Asp Ala Gln Ala Met Gly Gln Arg Pro Ile Thr
   65                  70                  75                  80
   Phe Leu Arg Gln Val Leu Ala Leu Cys Val Asn Pro Asp Leu Leu Ser
                   85                  90                  95
   Ser Pro Asn Phe Pro Asp Asp Ala Lys Lys Arg Ala Glu Arg Ile Leu
               100                 105                 110
   Gln Ala Cys Gly Gly His Ser Leu Gly Ala Tyr Ser Val Ser Ser Gly
           115                 120                 125
   Ile Gln Leu Ile Arg Glu Asp Val Ala Arg Tyr Ile Glu Arg Arg Asp
       130                 135                 140
   Gly Gly Ile Pro Ala Asp Pro Asn Asn Val Phe Leu Ser Thr Gly Ala
   145                 150                 155                 160
   Ser Asp Ala Ile Val Thr Val Leu Lys Leu Leu Val Ala Gly Glu Gly
                   165                 170                 175
   His Thr Arg Thr Gly Val Leu Ile Pro Ile Pro Gln Tyr Pro Leu Tyr
               180                 185                 190
   Ser Ala Thr Leu Ala Glu Leu Gly Ala Val Gln Val Asp Tyr Tyr Leu
           195                 200                 205
   Asp Glu Glu Arg Ala Trp Ala Leu Asp Val Ala Glu Leu His Arg Ala
       210                 215                 220
   Leu Gly Gln Ala Arg Asp His Cys Arg Pro Arg Ala Leu Cys Val Ile
   225                 230                 235                 240
   Asn Pro Gly Asn Pro Thr Gly Gln Val Gln Thr Arg Glu Cys Ile Glu
                   245                 250                 255
   Ala Val Ile Arg Phe Ala Phe Glu Glu Arg Leu Phe Leu Leu Ala Asp
               260                 265                 270
   Glu Val Tyr Gln Asp Asn Val Tyr Ala Ala Gly Ser Gln Phe His Ser
           275                 280                 285
   Phe Lys Lys Val Leu Met Glu Met Gly Pro Pro Tyr Ala Gly Gln Gln
       290                 295                 300
   Glu Leu Ala Ser Phe His Ser Thr Ser Lys Gly Tyr Met Gly Glu Cys
   305                 310                 315                 320
   Gly Phe Arg Gly Gly Tyr Val Glu Val Val Asn Met Asp Ala Ala Val
                   325                 330                 335
   Gln Gln Gln Met Leu Lys Leu Met Ser Val Arg Leu Cys Pro Pro Val
               340                 345                 350
   Pro Gly Gln Ala Leu Leu Asp Leu Val Val Ser Pro Pro Ala Pro Thr
           355                 360                 365
   Asp Pro Ser Phe Ala Gln Phe Gln Ala Glu Lys Gln Ala Val Leu Ala
       370                 375                 380
   Glu Leu Ala Ala Lys Ala Lys Leu Thr Glu Gln Val Phe Asn Glu Ala
   385                 390                 395                 400
   Pro Gly Ile Ser Cys Asn Pro Val Gln Gly Ala Met Tyr Ser Phe Pro
                   405                 410                 415
   Arg Val Gln Leu Pro Pro Arg Ala Val Glu Arg Ala Gln Glu Leu Gly
               420                 425                 430
   Leu Ala Pro Asp Met Phe Phe Cys Leu Arg Leu Leu Glu Glu Thr Gly
           435                 440                 445
   Ile Cys Val Val Pro Gly Ser Gly Phe Gly Gln Arg Glu Gly Thr Tyr
       450                 455                 460
   His Phe Arg Met Thr Ile Leu Pro Pro Leu Glu Lys Leu Arg Leu Leu
   465 470 475 480
   Leu Glu Lys Leu Ser Arg Phe His Ala Lys Phe Thr Leu Glu Tyr Ser
   485 490 495
<210> 14
   <211> 9
   <212> PRT
   <213> Homo sapiens
<400> 14
   Ile Asp Ala Ala Ser Pro Leu Glu Lys
   1 5
<210> 15
   <211> 15
   <212> PRT
   <213> Homo sapiens
<400> 15
   Phe Ser Leu Asp Ala Leu Ile Thr His Val Leu Pro Phe Glu Lys
   1 5 10 15
<210> 16
   <211> 12
   <212> PRT
   <213> Homo sapiens
<400> 16
   Ile Asn Glu Gly Phe Asp Leu Leu His Ser Gly Lys
   1 5 10
<210> 17
   <211> 8
   <212> PRT
   <213> Homo sapiens
<400> 17
   Ala Ala Val Leu Trp Glu Val Lys
   1 5
<210> 18
   <211> 9
   <212> PRT
   <213> Homo sapiens
<400> 18
   Ile Asp Asp Asp Ala Asn Leu Glu Arg
   1 5
<210> 19
   <211> 10
   <212> PRT
   <213> Homo sapiens
<400> 19
   Ile Ile Gly Ile Asp Ile Asn Ser Glu Lys
   1               5                   10
<210> 20
   <211> 15
   <212> PRT
   <213> Homo sapiens
<400> 20
   Phe Asn Leu Asp Ala Leu Val Thr His Thr Leu Pro Phe Asp Lys
   1               5                   10                  15
<210> 21
   <211> 9
   <212> PRT
   <213> Homo sapiens
<400> 21
   Glu Ala Thr Thr Glu Gln Gln Leu Lys
   1               5
<210> 22
   <211> 8
   <212> PRT
   <213> Homo sapiens
<400> 22
   Glu Ala Tyr Asn Leu Gly Val Arg
   1               5
<210> 23
   <211> 10
   <212> PRT
   <213> Homo sapiens
<400> 23
   Ala Ile Ala Glu Glu Leu Ala Pro Glu Arg
   1               5                   10
<210> 24
   <211> 11
   <212> PRT
   <213> Homo sapiens
<400> 24
   Thr Ile Gly Ile Ile Gly Ala Pro Phe Ser Lys
   1               5                   10
<210> 25
   <211> 11
   <212> PRT
   <213> Homo sapiens
<400> 25
   Asp Val Asp Pro Gly Glu His Tyr Ile Leu Lys
   1               5                   10
<210> 26
   <211> 11
   <212> PRT
   <213> Homo sapiens
<400> 26
   Glu Gly Leu Tyr Ile Thr Glu Glu Ile Tyr Lys
   1               5                   10
<210> 27
   <211> 11
   <212> PRT
   <213> Homo sapiens
<400> 27
   Ala Ile Gly Leu Pro Glu Glu Leu Ile Gln Lys
   1               5                   10
<210> 28
   <211> 8
   <212> PRT
   <213> Homo sapiens
<400> 28
   Phe Thr Ile Thr Ala Gly Ser Lys
   1               5
<210> 29
   <211> 10
   <212> PRT
   <213> Homo sapiens
<400> 29
   Thr Val Val Gln Leu Glu Gly Asp Asn Lys
   1               5                   10
<210> 30
   <211> 7
   <212> PRT
   <213> Homo sapiens
<400> 30
   Leu His Tyr Phe Asn Ala Arg
   1 5
<210> 31
   <211> 11
   <212> PRT
   <213> Homo sapiens
<400> 31
   Ser His Gly Gln Asp Tyr Leu Val Gly Asn Lys
   1 5 10
<210> 32
   <211> 9
   <212> PRT
   <213> Homo sapiens
<400> 32
   Ala Ile Leu Asn Tyr Ile Ala Ser Lys
   1 5
<210> 33
   <211> 8
   <212> PRT
   <213> Homo sapiens
<400> 33
   Ile Ser Asn Leu Pro Thr Val Lys
   1 5
<210> 34
   <211> 9
   <212> PRT
   <213> Homo sapiens
<400> 34
   Ala Leu Glu Leu Glu Gln Glu Leu Arg
   1 5
<210> 35
   <211> 10
   <212> PRT
   <213> Homo sapiens
<400> 35
   Leu Leu Val Ala Gly Glu Gly His Thr Arg
   1 5 10
<210> 36
   <211> 8
   <212> PRT
   <213> Homo sapiens
<400> 36
   Lys Pro Phe Thr Glu Val Ile Arg
   1               5